(19) Europäisches Patentamt
European Patent Office
Office européen des brevets

(11) Publication number: **0 247 482 B1**

(12) # EUROPEAN PATENT SPECIFICATION

(45) Date of publication of patent specification: **22.04.92**

(51) Int. Cl.5: **G01N 33/52**, //G01N31/22

(21) Application number: **87107161.9**

(22) Date of filing: **18.05.87**

(54) **Hardened reagent coatings, and processes for the preparation thereof.**

(30) Priority: **28.05.86 DE 3618035**
**18.07.86 DE 3624301**

(43) Date of publication of application:
**02.12.87 Bulletin 87/49**

(45) Publication of the grant of the patent:
**22.04.92 Bulletin 92/17**

(84) Designated Contracting States:
**DE FR GB IT**

(56) References cited:
**EP-A- 0 021 108**
**EP-A- 0 049 449**
**EP-A- 0 130 335**
**EP-A- 0 158 190**
**EP-A- 0 162 308**
**DD-A- 222 419**
**DE-A- 2 545 755**
**DE-A- 2 547 589**
**DE-A- 2 704 276**
**FR-A- 2 235 389**
**US-A- 3 321 313**

**PATENT ABSTRACTS OF JAPAN, vol. 7, no. 220 (P-226)[1365], 30 September 1983**

**PATENT ABSTRACTS OF JAPAN, vol. 6, no. 116 (P-125)[994], 29 June 1982**

(73) Proprietor: **MILES INC.**
**1127 Myrtle Street**
**Elkhart Indiana 46514(US)**

(72) Inventor: **Riebel, Alexander, Dr.**
**Höhenstr. 110**
**W-5090 Leverkusen 1(DE)**
Inventor: **Himmelmann, Wolfgang, Dr.**
**Im Ziegenfeld 7**
**W-5090 Leverkusen 3(DE)**
Inventor: **Sobel, Johannes, Dr.**
**Willi Baumeister-Str. 9**
**W-5090 Leverkusen 1(DE)**
Inventor: **Schranz, Karl, Dr.**
**Schillerstr. 1**
**W-5068 Odenthal(DE)**
Inventor: **Oosta, Gary, Dr.**
**22928 Bradford Court**
**Elkhart, IN 46516(US)**
Inventor: **White, William, Dr.**
**Clemens Brentano-Str. 13**
**W-4000 Düsseldorf 30(DE)**

(74) Representative: **Dänner, Klaus, Dr. et al**
**c/o Bayer AG Konzernverwaltung RP Patentabteilung**
**W-5090 Leverkusen 1 Bayerwerk(DE)**

EP 0 247 482 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to the use of hardened reagent coatings for clinical chemical measurements, and processes for the preparation of the coatings. The reagent coatings are so-called "dry-chemical" test agents, which are also known under the name test strips. The reagent coatings have a matrix, based on protein and/or polymer, which are hardened by carboxyl group-activating crosslinking agents, also named hardeners below. The reagent coatings useful according to the invention are so hard that they can be wiped without suffering damage.

When the reagent coatings according to the invention are brought into contact with the sample to be analysed, which is normally a biological liquid (for example blood, plasma, serum or urine), they absorb the sample, during which they swell to a pre-specified checkable extent.

In recent years, the introduction of "dry-chemical" test agents, also called test strips, has revolutionized and considerably simplified clinical-chemical measurements. In contrast to the "wet-chemical" methods, in which it is necessary to mix solutions and to measure the colour or flourescence change in sometimes very expensive measuring instruments, it is usually only necessary to bring the sample into contact with the test strip in the "dry-chemical" methods.

In some cases, previous dilution of the sample is necessary.

The literature is full of examples of "dry-chemical" test agents.

In all cases, the reagents and auxiliaries are contained in a carrier matrix. The sample which contains the substance to be determined is applied.

A reaction, which generates a measurable signal (for example colour or fluorescence), then occurs within the carrier matrix.

A large number of materials are known which are suitable as carrier matrix, such as, for example, filter paper (US Patents 3,092,463; 4,279,993; 4,318,985; 4,361,648; 4,273,868; 4,318,709; 4,190,419; 4,215,157; 4,038,031) or proteins of natural origin (US Patents 3,983,005; 4,042,335; 4,098,594; 4,166,093; 4,274,832; 4,255,384; 4,452,887; 4,340,565).

The use of synthetically prepared polymers is also described (US Patents 3,630,957; 4,312,834).

The measurement of the blood sugar content in whole blood is particularly important in the treatment of diabetes. If the patient is able to carry out such an analysis himself, he has the opportunity of carrying out his own therapy check, and the drug therapy and also diet can be monitored and adjusted better. It is required of such a test that it is simple to carry out, ie. the sample (for example whole blood) is applied to the test strip and the excess of sample is removed after a pre-specified time by wiping or swabbing.

One of the prerequisites that such a test agent must fulfil is that the matrix (reagent coating(s)) may only take up a particular and reproducible amount of sample liquid. In addition, the matrix must be so hard physically that it cannot be damaged by the wiping or swabbing of the sample excess.

In the search for a suitable carrier matrix which fulfils these conditions, the starting point was a water-soluble polymer which is hardened by crosslinking substances during the preparation of the reagent coatings. Gelatin, for example, was used as soluble polymer.

Most of the known and tested crosslinking agents are unusable for the preparation of reagent coatings.

Some react with the indicator dyestuffs and thereby lead to colour changes. Others liberate mineral acids during the reaction or deactivate the enzymes which are necessary for the detection reaction. Still others lead to inflexible coatings or to coatings having inadequate abrasion resistance. Some react too quickly or too slowly or are too unstable in an aqueous environment.

Surprisingly, Crosslinking agents have now been found which have the properties required for reagent coatings.

The present invention relates to the use of hardened reagent coatings for clinical chemical measurements, having a matrix based on polymer and/or protein, which are hardened using a carboxyl group-activating crosslinking agent. Carboxyl group-activating crosslinking agents are those crosslinking agents which react, in an initial reaction, with carboxyl groups of the polymer or protein functioning as binding agent and thus activate the carboxyl groups.

These activated carboxyl groups are capable of further reacting with other functional groups, such as, for example, amino groups, SH groups or hydroxyl groups, of the binding agent, thus causing the crosslinking of the matrix.

Suitable binding agents (matrix materials) are proteins and derivatives thereof which naturally contain carboxyl groups which can be activated. Proteins also contain the other functional groups which are necessary for the crosslinking.

Gelatin, gelatin derivatives, casein, or also zein, should be mentioned as suitable proteins, gelatin and the derivatives thereof being preferred. Polymers, for example ampholytic polymers, which contain the

abovementioned groups are likewise suitable as binding agents (matrix material).

The hardened reagent coatings are coating which further contain reagents from the group comprising enzymes, coenzymes, effectors, indicators, substrates, antigens, antibodies, stabilisers, wetting agents and buffers.

The crosslinking agent can be added in an amount of 0.2 to 40 % by weight, relative to the weight of the binding agent.

Applied is the crosslinking agent by coating with a 0.2 to 5 % strength solution of the crosslinking agent wherein the solution is an alcohlolic, agueous or aqueous-alcoholic solution.

Carboxl group-activating crosslinking agents which are suitable for the present invention are carbamoylammonium compounds of the general formula I

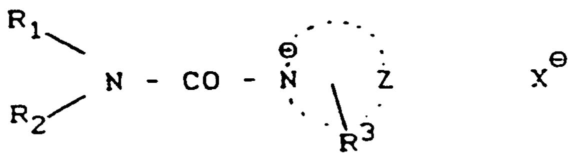

in which:

$R_1$ denotes an alkyl group, preferably having 1-3 C atoms, an aryl group which is optionally substituted by a $C_1$ - $C_3$ alkyl radical or by halogen, for example phenyl which is optionally substituted by methyl, ethyl or propyl, Cl or Br, or denotes an aralkyl group, for example benzyl, which may be substituted in the same fashion as the aryl group,

$R_2$ has the same meaning as $R_1$ or represents a divalent, optionally substituted alkylene, arylene, aralkylene or alkyl-aryl-alkyl radical, for example an ethylene, propylene, phenylene or xylylene radical, which is bonded to a further carbamoylammonium group of the above general formula via its second bond, or

$R_1$ and $R_2$, together, form the group of atoms which is necessary for completion of an optionally substituted piperidine, piperazine or morpholine ring, it being possible for the ring to be substituted, for example by an alkyl group having 1-3 C atoms or by halogen, such as Cl or Br,

| | |
|---|---|
| $R_3$ denotes - $NR_4$-CO-$R_5$ | $R_4$ denotes H or alkyl (1-4 C)<br>$R_5$ denotes H, alkyl (1-4 C) or $NR_6R_7$, and<br>$R_6$ and $R_7$ denote H or alkyl ($C_1$-$C_4$) |
| $R_3$ denotes - $(CH_2)m$ - $NR_8R_9$ | $R_8$ denotes -CO-$R_{10}$<br>$R_9$ denotes H or alkyl ($C_1$-$C_4$)<br>$R_{10}$ denotes $NR_{11}R_{12}$<br>$R_{11}$ denotes alkyl ($C_1$-$C_4$) or aryl<br>$R_{12}$ denotes H, alkyl or aryl, and<br>m denotes 1-3 |
| $R_3$ denotes - $(CH_2)_n$ - $CONR_{13}R_{14}$ | $R_{13}$ denotes H, alkyl ($C_1$-$C_4$) or aryl<br>$R_{14}$ denotes H or alkyl ($C_1$-$C_4$), or<br>$R_{13}$ and $R_{14}$ together form the group of atoms which is necessary for completion of a 5- or 6-membered aliphatic ring and<br>n denotes 0-3 |
| $R_3$ denotes | 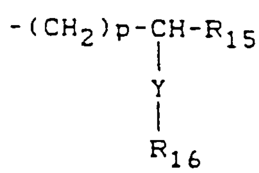<br>$R_{15}$ denotes H or alkyl ($C_1$-$C_4$) which is optionally substituted by halogen<br>Y denotes -O- or -$NR_{17}$-,<br>$R_{16}$ denotes H, alkyl or -CO-$R_{18}$ or -CO-$NHR_{19}$<br>$R_{17}$, $R_{18}$ and $R_{19}$ denote H or alkyl ($C_1$-$C_4$) and<br>p denotes 2-3 |

Z denotes the C atoms which are necessary for completion of a 5- or 6-membered aromatic heterocyclic ring, optionally with fused benzene ring, and
$X^{\ominus}$ denotes an acid anion.

Carbamoylammonium compound of the general formula II

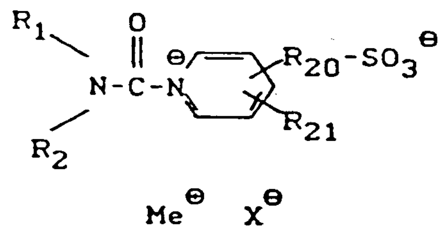

in which
$R_1$ and $R_2$ have the meaning stated in the case of the carbamoylammonium compounds of the formula I and
$R_{20}$ represents methylene, ethylene, propylene or a single chemical bond,
$R_{21}$ represents hydrogen, methyl or ethyl,
$X^{\ominus}$ represents an acid anion, such as halogen$^{\ominus}$, $BF_4{}^{\ominus}$, $NO_3{}^{\ominus}$, $SO_4{}^{\ominus}$, $ClO_4{}^{\ominus}$ or $CH_3OSO_3{}^{\ominus}$, and
$Me^{\oplus}$ represents an alkali metal cation,
should be mentioned as being preferred.

Compounds of the general formula III and IV are furthermore suitable as crosslinking agents.

In the compounds of the formula III

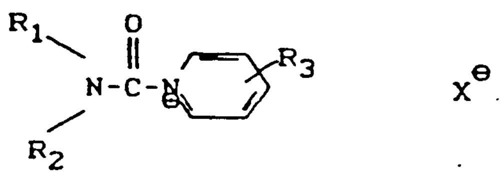

$R_1$ and $R_2$ (which may be identical or different) in each case denote an alkyl group having 1 to 10 carbon atoms (for example methyl, ethyl, 2-ethylhexyl ), an aryl group having 6 to 15 carbon atoms (for example phenyl, naphthyl ), or an aralkyl group having 7 to 15 carbon atoms (for example benzyl, phenethyl ), or they may be combined with one another to form a heterocyclic ring together with the nitrogen atom (for example a pyrrolidine ring, a piperazine ring, a morpholine ring ); $R_3$ denotes a hydrogen atom, a halogen atom, a carbamoyl group, a sulpho group, a halogen atom, a carbamoyl group, a sulpho group, a ureido group, an alkoxy group having 1 to 10 carbon atoms, an alkyl group having 1 to 10 carbon atoms etc. When $R_3$ represents an alkyl or an alkoxy group, as described above, these groups may in each case be further substituted by a halogen atom, a carbamoyl group, a sulpho group, a ureido group etc. In the above formula, $X^-$ represents an anion and acts as counterion for the N-carbamoylpyridinium ion with formation of a salt, or it may be absent therein when $R_3$ contains a sulpho group as component, and an intramolecular salt is thus formed. Suitable examples of an anion represented by $X^-$ comprise halide ions, the sulphonic acid ion, sulphonate ion, $ClO_4{}^-$, $BF_4{}^-$, $PF_6{}^-$ , and also carbamoyloxoammonium compounds of the formula IV

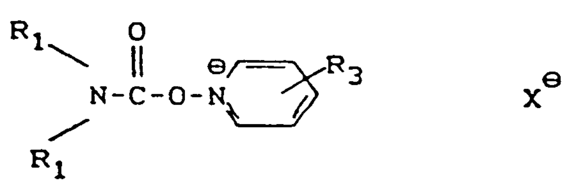

in which

$R_1$, $R_2$, $R_3$ and $X^-$ have the same meanings as stated in the formula III.

In the crosslinking agents of the formula V

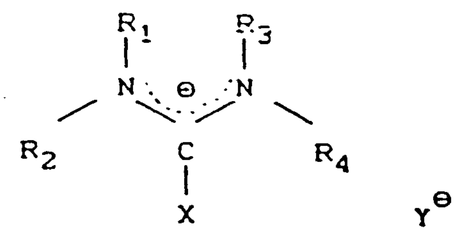

$R_1$, $R_2$, $R_3$ and $R_4$ (which may be identical or different) in each case denote an alkyl group having 1 to 20 carbon atoms (for example methyl, ethyl, butyl, 2-ethylhexyl, dodecyl ), an aralkyl group having 6 to 20 carbon atoms (for example phenyl, phenethyl, 3-pyridylmethyl ), or an aryl group having 5 to 20 carbon atoms (for example phenyl, naphthyl, pyridyl, ). These groups may in each case contain a substituent group (for example a halogen atom, a sulpho group, an alkoxy group having 1 to 20 carbon atoms, an alkoxy group having 6 to 20 carbon atoms, an N,N-disubstituted carbamoyl group ).

It is also possible that any two groups, selected from $R_1$, $R_2$, $R_3$ and $R_4$, are combined with one another to form a ring. For example, it is possible that $R_1$ and $R_2$ or $R_3$ and $R_4$ are combined with one another to form a ring together with a nitrogen atom. Specific examples of a ring formed in such a case comprise a pyrrolidine ring, a piperazine ring, a perhydroazepine ring, a morpholine ring, etc. In another case, $R_1$, and $R_3$ or $R_2$ and $R_4$ are combined with one another to form a ring together with two nitrogen atoms, and a carbon atom located between them. Specific examples of a ring formed in such a fashion comprise an imidazoline ring, a tetrahydropyrimidine ring, a tetrahydrodiazepine ring etc.

In the formula V, X denotes a group which can be cleaved off from the compound of the formula V by reaction with a nucleophilic reagent, suitable examples comprising a halogen atom, a sulphonyloxy group, a 1-pyridiniumyl group . In the above formula V, $Y^-$ denotes an anion, suitable examples comprising a halide ion, a sulphonate ion, a sulphuric acid ion, $ClO_4^-$, $BF_4^-$, $PF_6^-$ .

When any one of $R_1$, $R_2$, $R_3$ and $R_4$ has a sulpho group, a crosslinking agent of the formula V can form an intramolecular salt without independent counterion, which is represented by $Y^-$.

In the carbodiimides of the formula VI

$R_1 - N = C = N - R_2$

which are suitable as crosslinking agents, $R_1$ denotes an alkyl group having 1 to 10 carbon atoms (for example methyl, ethyl, 2-ethylhexyl ), a cycloalkyl group having 5 to 8 carbon atoms (for example cyclohexyl ), an alkoxyalkyl group having 3 to 10 carbon atoms (for example methoxyethyl ), or an aralkyl group having 7 to 15 carbon atoms (for example benzyl, phenethyl ). In the above formula VI, $R_2$ is favourably a group of the following formula in addition to the groups defined as $R_1$:

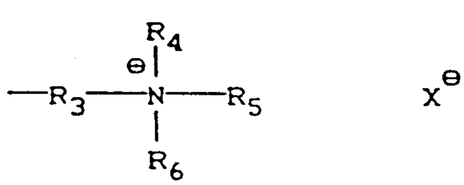

in which $R_3$ represents an alkylene group having 2 to 4 carbon atoms (for example ethylene, propylene, trimethylene ): and $R_4$ and $R_4$ (which may be identical or different) in each case denote an alkyl group having 1 to 6 carbon atoms (for example methyl, ethyl, .), or it is also favourable when they are combined with one another to form a heterocyclic ring together with a nitrogen atom (for example pyrrolidine, piperazine, morpholine ). In the above formula, $R_6$ denotes an alkyl group having 1 to 6 carbon atoms (for example methyl, ethyl, butyl .), which is preferably substituted by a certain substituent. Suitable examples of such substituents comprise unsubstituted and substituted carbamoyl groups and the sulpho group. $X^-$ denotes an anion, suitable examples comprising halide ions, sulphonate ions, sulphuric acid ions, $ClO_4^-$, $BF_4^-$, $PF_6^-$ . When the hardener of the formula V forms an intramolecular salt, the counteranion

represented by X is not necessary.

In the halogenopyridinium compounds of the formula VII

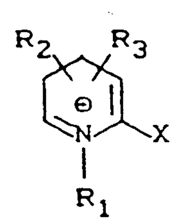

$Y^{\ominus}$

$R_1$ denotes an alkyl group having 1 to 10 carbon atoms (for example methyl, ethyl, butyl ), an aryl group having 6 to 15 carbon atoms (for example phenyl, naphthyl ), or an aralkyl group having 7 to 15 carbon atoms (for example benzyl, phenethyl, ). These groups may in each case be substituted by any substituent. Suitable examples of such substituents comprise a carbamoyl group, a sulphamoyl group, a sulpho group, etc. In the formula VII, $R_2$ and $R_3$ (which may be identical or different) in each case represent a hydrogen atom a halogen atom, an acylamido group, a nitro group, a carbamoyl group, a ureido group, an alkoxy group, an alkyl group, an alkenyl group, an aryl group, an aralkyl group . In addition, it is also favourable when $R_2$ and $R_3$ are combined with one another to form a condensed ring together with the pyridinium ring structure.

In the formula VII, X denotes a group which is cleaved off from the compound represented by the formula VII by reaction with a nucleophilic reagent, preferred examples being halogen atoms, the sulphonyloxy group, or groups represented by

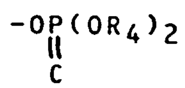 or 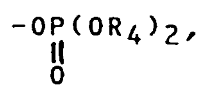,

in which $R_4$ represents an alkyl group or an aryl group. When X is a sulphonyloxy group, it is also favourable for X to be bonded to $R_1$.

In the formula VII, $Y^-$ represents an anion, preferred examples comprising halide ions, sulphonate ions, sulphuric acid ions, $ClO_4^-$, $BF_4^-$, $PF_6^-$ . However, $Y^-$ may be absent when $R_1$ is a sulphosubstituted group, whereby the compound forms an intramolecular salt.

In the sulphonylaminopiperidinium salts of the formula VIII

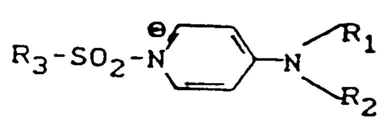

$X^{\ominus}$

$R_1$ and $R_2$ have the same meanings as defined for $R_1$ and $R_2$ in the formula III, and $R_3$ denotes an alkyl group having 1 to 10 carbon atoms (for example methyl, ethyl, butyl, ), an aryl group having 6 to 15 carbon atoms (for example phenyl, naphthyl, ), or an aralkyl group having 7 to 15 carbon atoms (for example benzyl, phenylethyl, ). $X^-$ denotes an anion, preferred examples comprising halide ions; sulphonate ions, sulphuric acid ions, $ClO_4^-$, $BF_4^-$, $PF_6^-$.

In addition to the hardeners represented by the above formulae I to VIII, compounds, described in JP-OS (Japanese Published Specifications) 38,540/75, 93,470/77, 43,353/81 and 113,929/83 and in US Patent Specification 3,321,313, are also advantageously used as hardeners of the carboxyl group-activating type.

Specific examples of hardeners of the type described above which can particularly advantageously be used are illustrated below with reference to examples, but without representing a limitation.

a) Compounds of the formulae I-III

1

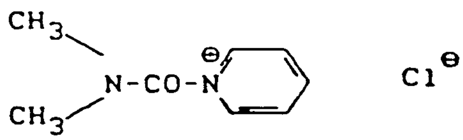

Syrup, very hygroscopic

2

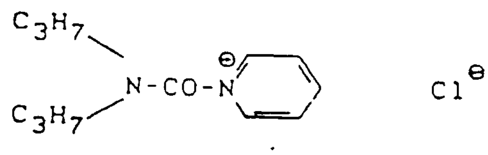

Syrup, very hygroscopic

3

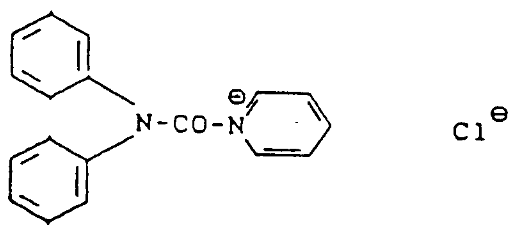

m.p. 112°C

4

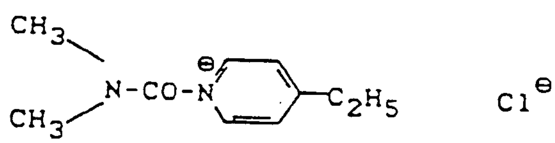

m.p. 103°C

5

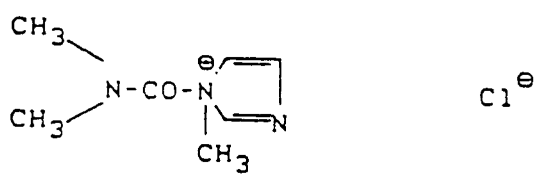

m.p. 87-89°C

6

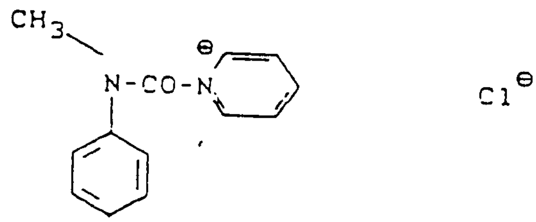

m.p. 108-110°C

7

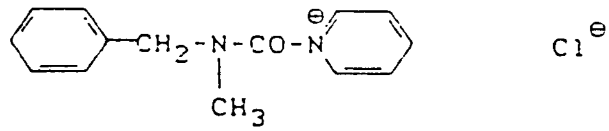

Syrup, hygroscopic

8

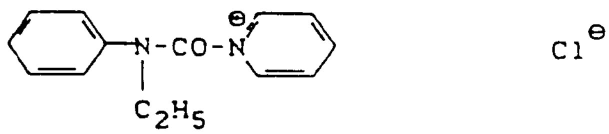

m.p. 105-107°C

9

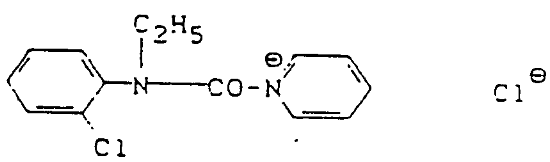

Syrup

10

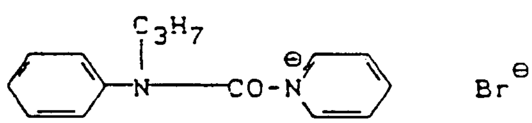

m.p. 103-105°C

11

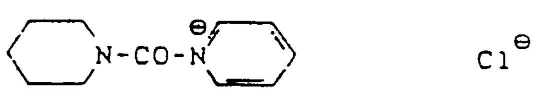

m.p. 75-77°C

12

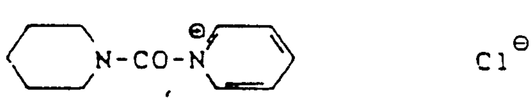

m.p. 110-112°C

13 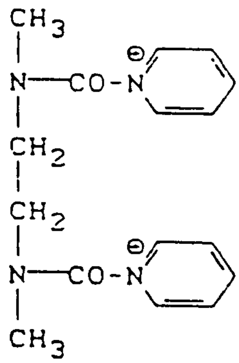 $Cl^{\ominus}$ $Cl^{\ominus}$

m.p. 95-96°C

14 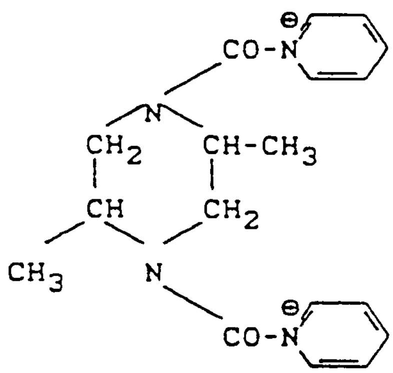 $Cl^{\ominus}$ $Cl^{\ominus}$

m.p. 106°C

15 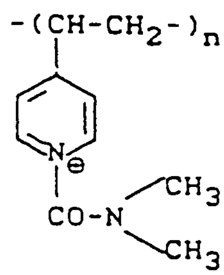 molecular weight greater than 10,000

16 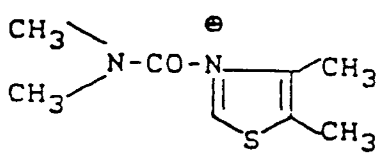 $Cl^{\ominus}$

m.p. 66-68°C

17 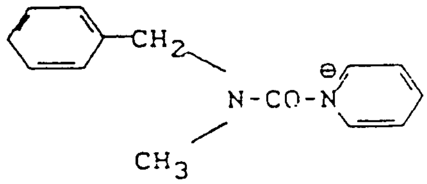 $Cl^{\ominus}$

18 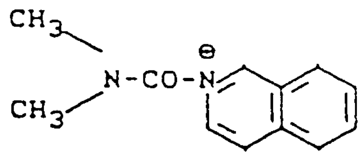 $Cl^{\ominus}$

Oil

Compound

19 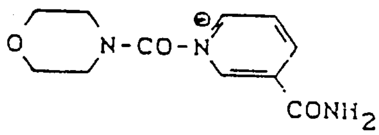 $Cl^{\ominus}$ Temp.: 103-105°C

20 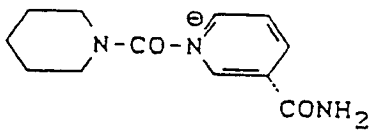 Oil

21 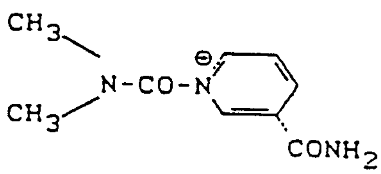 $Cl^{\ominus}$ Temp.: 109°C

22 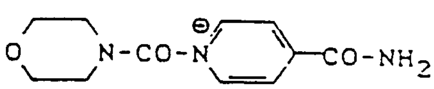 $ClO_3^{\ominus}$

23 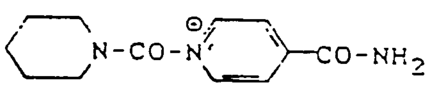 $Cl^{\ominus}$ Oil

Compound

24 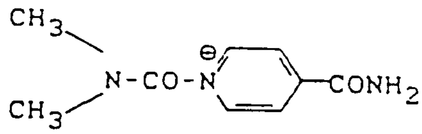 $Cl^{\ominus}$ Temp.: 115°C

25 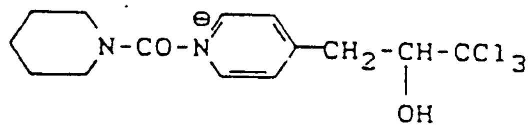 $Cl^{\ominus}$ Temp.: 154°C

26 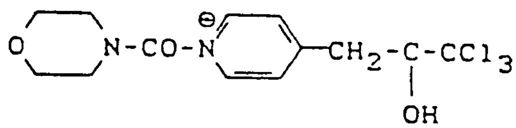 $Cl^{\ominus}$ Temp.: 140°C

27 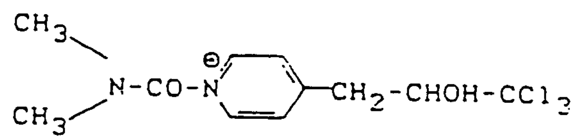 $Cl^{\ominus}$ Temp.: 115°C

28 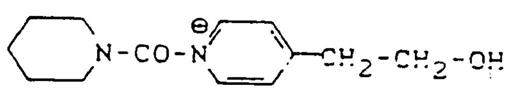 $Cl^{\ominus}$

29 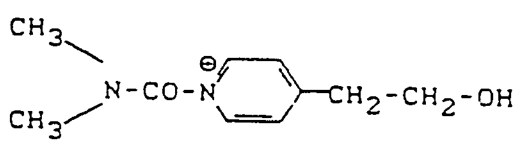 $Cl^{\ominus}$ Temp.: 140°C

30 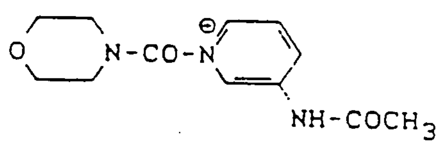 $Cl^{\ominus}$ Temp.: 118-120°C

31 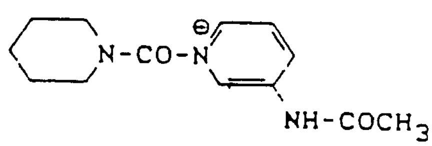 $Cl^{\ominus}$ Temp.: 90°C

Compounds

32 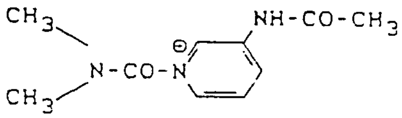 Cl$^{\ominus}$ Temp.: 210°C

33 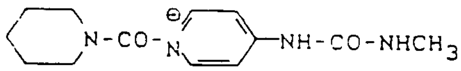 Cl$^{\ominus}$ Oil

34 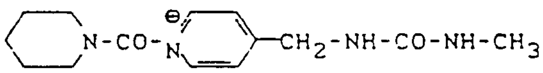 $BF_4^{\ominus}$ Oil

35 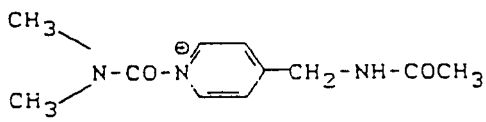 Cl$^{\ominus}$ Oil

36 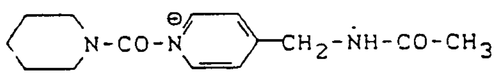 Cl$^{\ominus}$ Oil

37 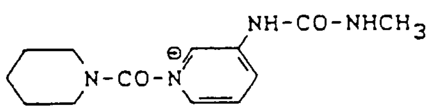 Cl$^{\ominus}$ Temp.: 60-65°C

38 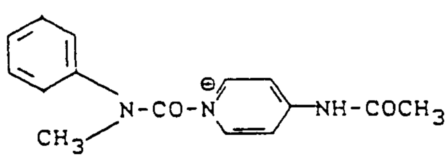 Cl$^{\ominus}$

39 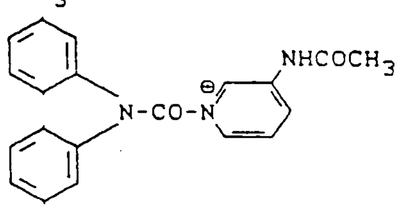 Cl$^{\ominus}$

40 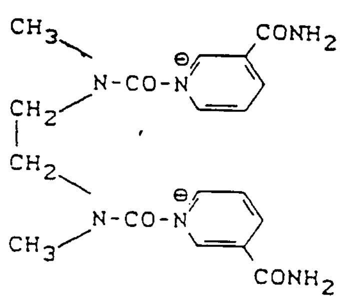 Cl$^{\ominus}$ Cl$^{\ominus}$

41 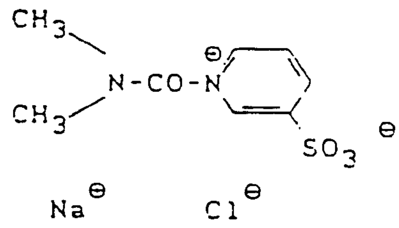

42 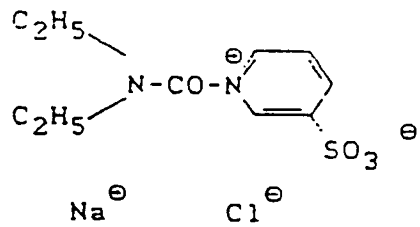

43 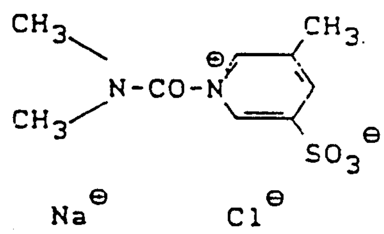

44 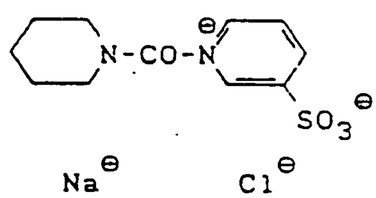

45 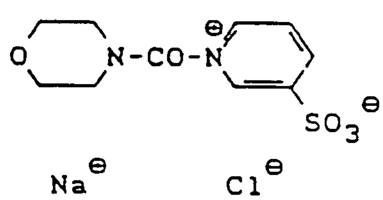

46 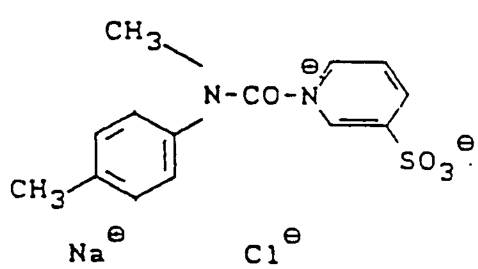

47

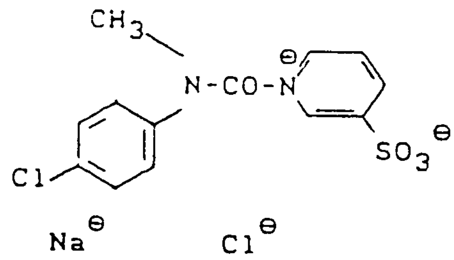

48

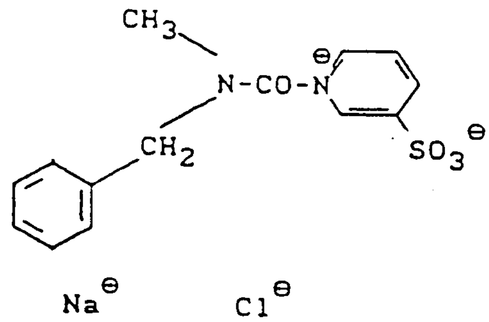

49

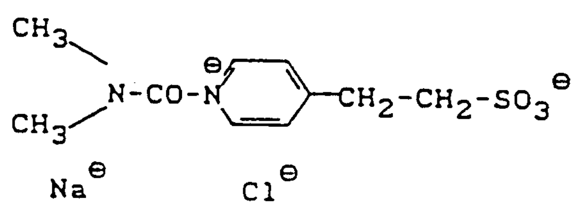

50

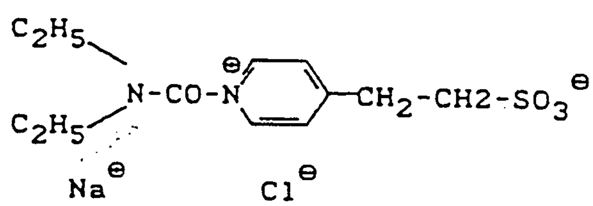

51

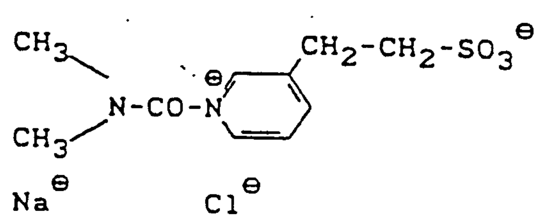

52 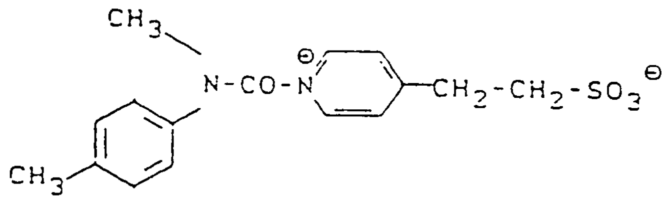

$Na^{\ominus}$ $Cl^{\ominus}$

53 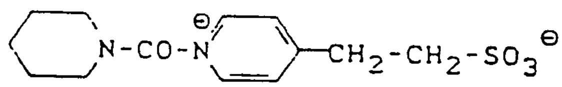

$Na^{\ominus}$ $Cl^{\ominus}$

54 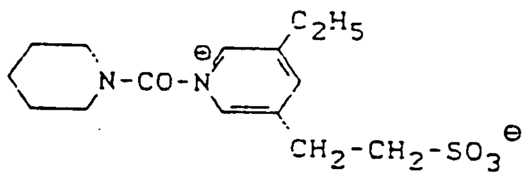

$Na^{\ominus}$ $Cl^{\ominus}$

55 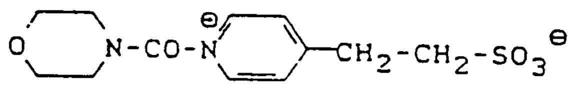

$Na^{\ominus}$ $Cl^{\ominus}$

56 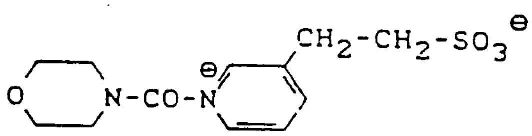

$K^{\ominus}$ $Cl^{\ominus}$

57 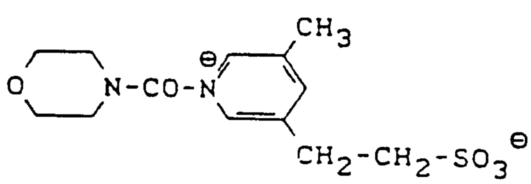

$Na^{\ominus}$ $Cl^{\ominus}$

58 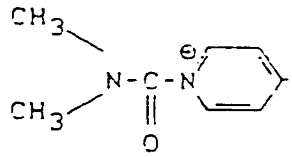 $PF_6^{\ominus}$

59 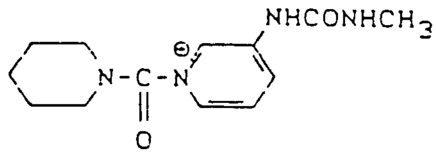 $Cl^{\ominus}$

60 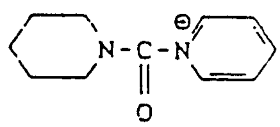 $Cl^{\ominus}$

61 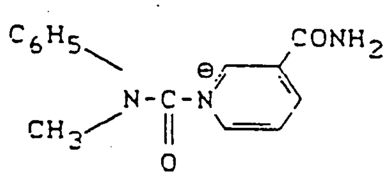 $BF_4^{\ominus}$

62 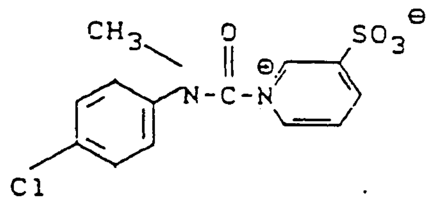

63 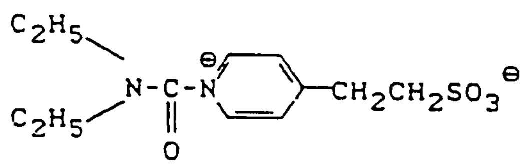

64 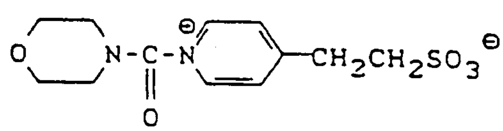

65 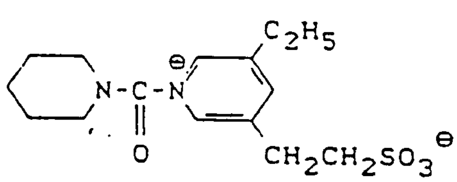

The compounds can be produced in a simple fashion which is known from the literature. The carbamoyl chlorides, which are then reacted with aromatic, heterocyclic nitrogen-containing compounds in the dark, are prepared from the secondary amines, for example using phosgene. The preparation of compound 3 is described in Chemischen Berichten 40, (1907), page 1831.

Further information on the synthesis is also located in JP-OS (Japanese Published Specification) 51,945/74 and JP-OS (Japanese Published Specification) 59,625/76.

b) Compounds of the formula IV

66 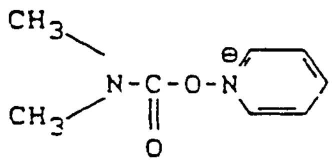 $Cl^{\ominus}$

67 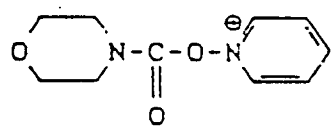 $Cl^{\ominus}$

68 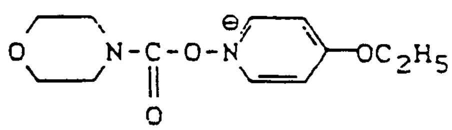 $ClO_4^{\ominus}$

69 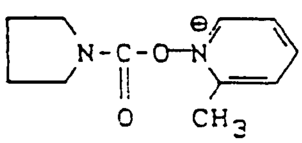 $Cl^{\ominus}$

70 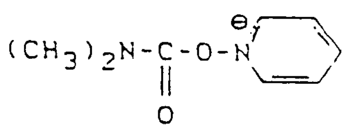 $Cl^{\ominus}$

c) Compounds of the formula V

Methods for the synthesis of these compounds are described in more detail in Chemistry Letters (The Chemical Society of Japan), page 1891-1894 (1982).

| | | |
|---|---|---|
| 71 | 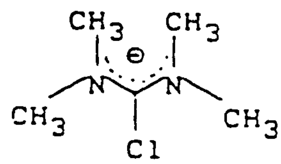 | $Cl^{\ominus}$ |
| 72 | 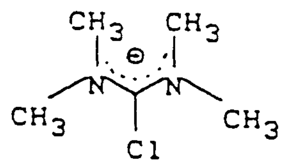 | $PF_6^{\ominus}$ |
| 73 | 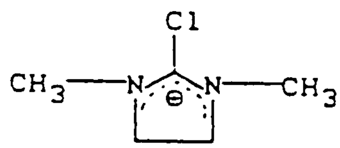 | $PF_6^{\ominus}$ |
| 74 | 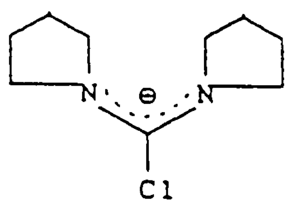 | $BF_4^{\ominus}$ |
| 75 | 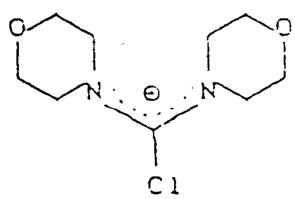 | $Cl^{\ominus}$ |
| 76 | 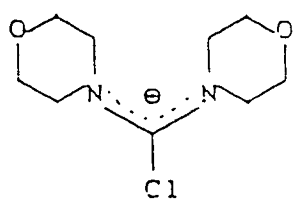 | $BF_4^{\ominus}$ |
| 77 | 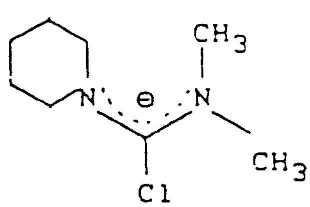 | $PF_6^{\ominus}$ |
| 78 | 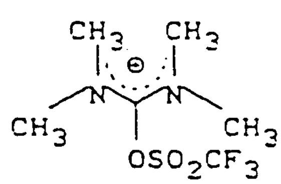 | $CF_3SO_3^{\ominus}$ |

d) Compounds of the formula VI

Methods for the synthesis of these compounds are described in more detail in JP-OS (Japanese Published Specifications) 126,125/76 and 48,311/77.

79 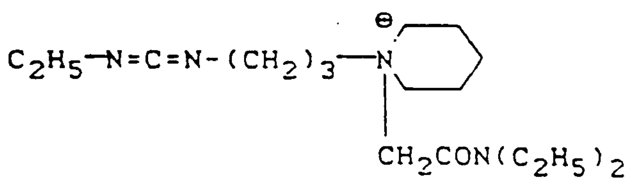 $Cl^{\ominus}$

80 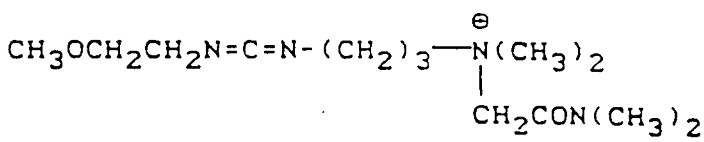 $Cl^{\ominus}$

81 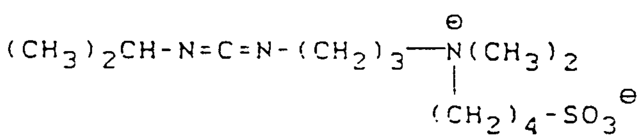 $Cl^{\ominus}$

82 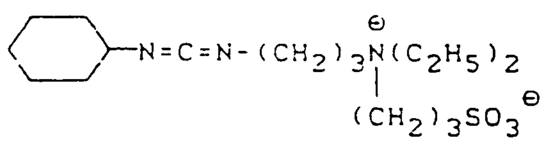

83 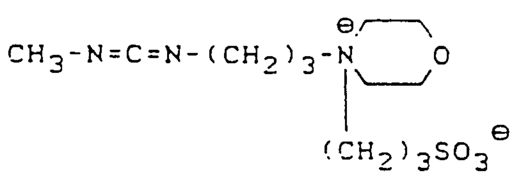

84 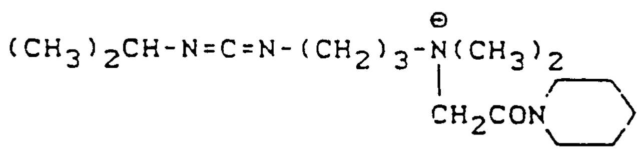 $Cl^{\ominus}$

85 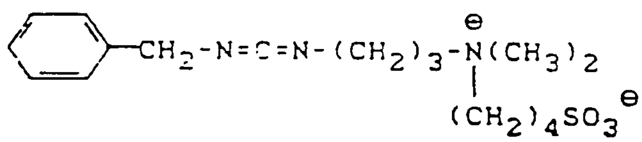

e) Compounds of the formula VII

Methods for the synthesis of these compounds are described in more detail in JP-OS (Japanese Published Specifications) 44,140/82 and 46,538/82, and in Japanese Patent Specification 50,669/83.

86 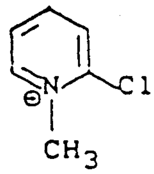 $ClO_4^{\ominus}$

87 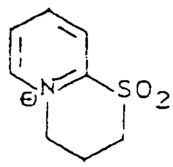 $Cl^{\ominus}$

88 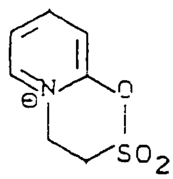 $Cl^{\ominus}$

89 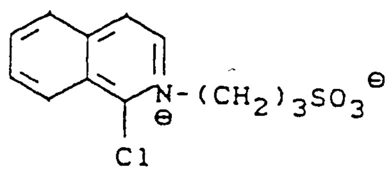

90 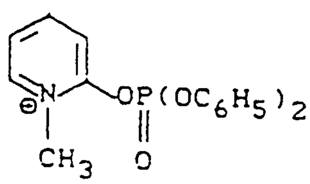 $Cl^{\ominus}$

91 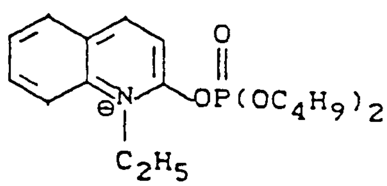 $I^{\ominus}$

f) Methods of the formula VIII

Method for the synthesis of these compounds are described in more detail in JP-OS (Japanese Published Specification) 54,427/77.

92 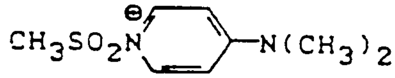 $Cl^{\ominus}$

93 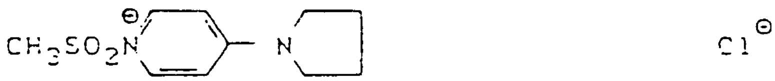

94 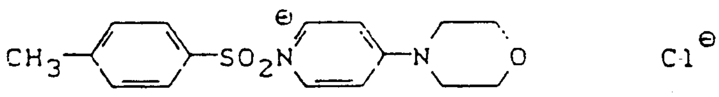

g) Compounds, described in JP-OS (Japanese Published Specification) 38,540/75

95 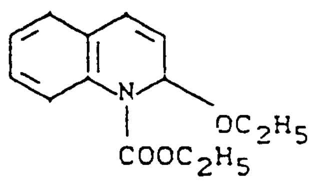

96 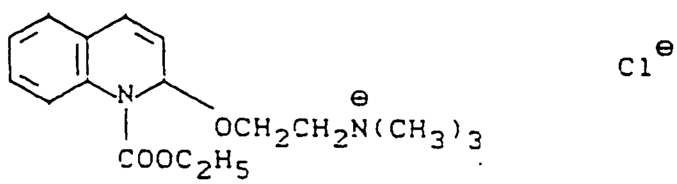

97 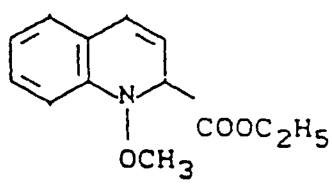

h) Compounds, described in JP-OS (Japanese Published Specification) 93,470/77

98 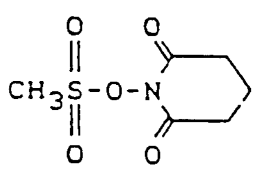

99 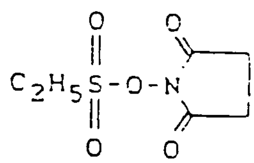

i) Compounds, described in JP-OS (Japanese Published Specification) 43,353/81

100

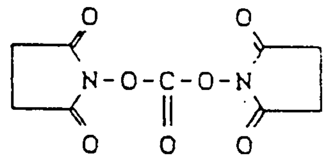

101

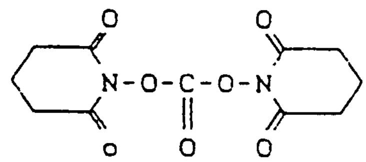

102

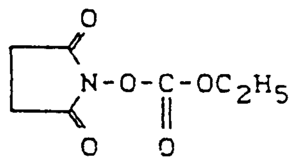

j) Compounds, described in JP-OS (Japanese Published Specification) 113,929/83

103

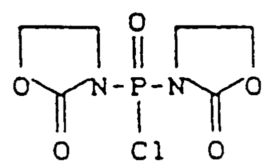

104

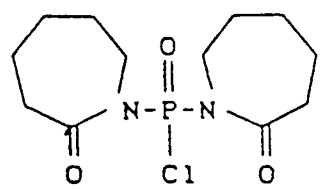

k) Compounds described in US Patent Specification 3,321,313

105 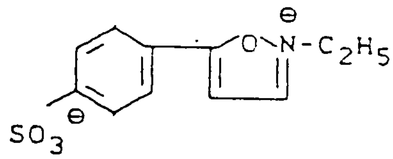

106 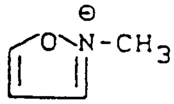 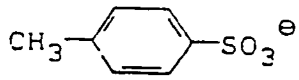

107 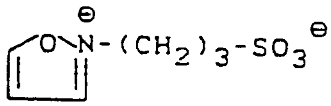

108 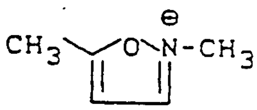 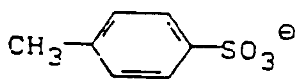

109 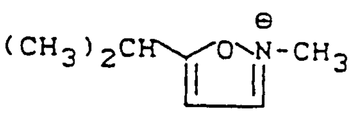 $ClO_4^{\ominus}$

110 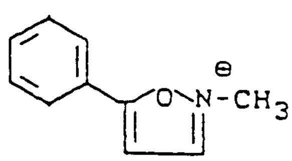 $ClO_4^{\ominus}$

111 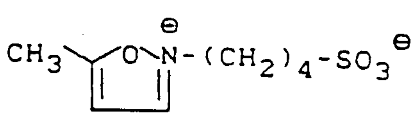

The synthesis of the crosslinking agents is described in the following examples, with the example of some of the compounds mentioned.

<u>Example 1</u> (Compound 6)

Phenyl-methyl-carbamoyl chloride

49.5 g of phosgene are passed carefully into 400 $cm^3$ of absolute toluene with cooling (22-25 °C). A solution of 107 g of distilled N-methylaniline in 450 $cm^3$ of absolute toluene is added dropwise to this solution with vigorous stirring. The mixture is subsequently heated at 80-90 °C for half an hour and cooled, and the precipitated hydrochloride is filtered off under suction. The toluene solution is finally evaporated to

dryness in vacuo with exclusion of moisture. The residue crystallizes on cooling.
Yield: 81 g, melting point 85 °C.

Compound 6:

33.9 g of phenyl-methyl-carbamoyl chloride are stirred into 400 $cm^3$ of absolute pyridine in a darkened room with exclusion of moisture. The carbamoylpyridinium chloride formed precipitates after 3 hours. After addition of an equal amount of ether, the crystals are filtered off under suction. Taken up in a little absolute ethanol and again precipitated using ether. The compound is filtered off under suction, washed well with ether and dried in a desiccator.
Yield: 41 g
Melting point 108-110 °C.

Example 2 (Compound 15

Compound 15, corresponding to poly-4-vinyl-NN-dimethylcarbamoylpyridinium chloride, is prepared as follows:
10.5 g of poly-4-vinyl-pyridine (molecular weight 10,000) are dissolved in
150 ml of absolute ethanol, and
10.8 g of diemthylcarbamoyl chloride are added with vigorous stirring.

The mixture is stirred at room temperature for 5 hours, and concentrated in vacuo. The mixture is passed under stirring, into ether, and the precipitated product is filtered off under suction. It is washed well with ether.

The compound is dried in a desiccator.
Yield: 18 g.

Example 3 (Compound 30)

1/20 mol of morpholinocarbonyl chloride are added, with stirring, to a solution of 1/20 mol of 3-acetaminopyridine in 60 ml of chloroform. The mixture is stirred for a further 2 hours at 50 °C with exclusion of moisture. The chloroform is then removed in vacuo and the highly-viscous residue is triturated with a little absolute acetone. The crystalline product is filtered off under suction and washed with a little absolute ether. The product is then dried in a desiccator.
Yield: 10 g
Melting point 118-123 °C.

Example 4 (Compound 41)

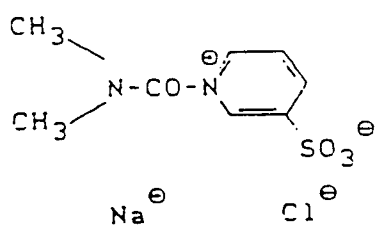

15.9 g of pyridinesulphonic acid are slurried in 200 ml of absolute dimethylformamide. A solution of 2.6 g of sodium in 150 ml of methanol is added dropwise to this (pH 7). The solution is filtered, and 13 g of N,N-dimethylcarbamoyl chloride are added. The mixture then remains standing at room temperature overnight. The reaction product precipitates after addition of 400 ml of ether. It is filtered off under suction and washed thoroughly with ether.
Yield: 19.5 g
Decomposition point: 250 °C

Example 5 (Compound 44)

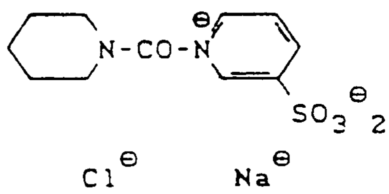

18 g of the sodium salt of pyridine-3-sulphonic acid are dissolved in a mixture of 1000 ml of dimethylformamide (dry) and 100 ml of methanol, and 15 g of piperidinocarbonyl chloride are added. The mixture remains standing overnight and the reaction product is precipitated using 200 ml of ether. It is filtered off under suction and washed with ether.
Yield: 13 g
Decomposition point = above 250 ° C.

Example 6 (Compound 45)

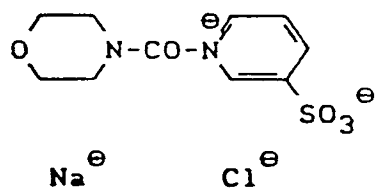

15.9 g of pyridine-3-sulphonic acid are dissolved in 200 ml of absolute dimethylformamide, and a solution of 2.6 g of sodium in 150 ml of absolute methanol is added dropwise (at pH 7).

The solution obtained was separated from a small amount of insoluble components by filtration, and then reacted with 18.9 g of morpholinylcarbonyl chloride. The mixture remained standing at room temperature for 24 hours. The reaction product was partly precipitated. The yield was improved by addition of absolute ether.

The residue is filtered off under suction and washed with absolute ether.
Yield: 18 g
Melting point: 236-237 ° C

Example 7 (Compound 46)

Preparation of compound 46:

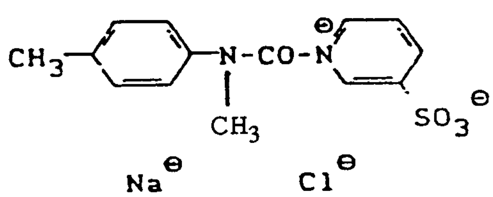

18.2 g of the sodium salt of pyridine-3-sulphonic acid are dissolved in a mixture of 100 ml of methanol and 100 ml of dimethylformamide. 18.5 g of N-methyl-N-methylphenylcarbonyl chloride are added to the mixture. After standing for 3 days, few crystals precipitate. They are filtered off under suction and washed with ether.
Yield: 3 g
Melting point : above 300 ° C.

Example 8 (Compound 55)

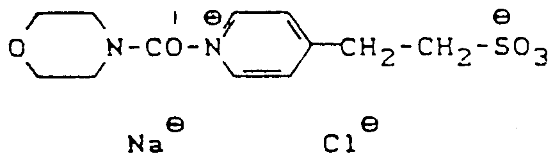

18.9 g of the sodium salt of pyridine-4-ethanesulphonic acid are dissolved in a solution of 400 ml of dimethylformamide (dry) and 400 ml of methanol (dry). 15 g of morpholinylcarbonyl chloride are added to this. After standing overnight, the reaction product is precipitated using 1.3 litres of ether. The crystals are filtered off under suction and washed with ether.
Yield: 22 g
Melting point: 152-153 ° C.

| Analysis: | | |
|---|---|---|
| | Calc.: | Found: |
| C | 40.1 % | 39.6 % |
| H | 4.5 % | 4.7 % |
| Cl | 9.9 % | 10.1 % |
| N | 7.8 % | 7.2 % |
| S | 8.9 % | 9.3 % |
| Na | 6.4 % | 6.2 % |

The odour threshold of pyridine is around 0.0004 mg/$m^3$ of air, so the perceptibility of pyridine using the sense of smell starts at very low quantities. Compared to this, the maximum workplace concentration for pyridine, its MWC, is considerably higher, namely at 15 mg/$m^3$. The smell test is therefore a thoroughly suitable means for determining very low pyridine concentrations at the workplace. The workplace concentration of pyridine must not exceed the limit 15 mg/$m^3$ of air (Reichhard, Lösungsmitteleffekte in der organischen Chemie [Effects of Solvents in Organic Chemistry], Verlag Chemie, page 172).

The compounds used as hardening agents according to the present invention are odourless, since they are fixed in the coating. This can be seen in a simple fashion by evaporating about 1 ml of a 5 % strength aqueous solution of a compound to dryness and checking this process by smell tests to a pyridine-like odour. When used for hardening reagent coatings corresponding to the present processes, the compound is subjected to similar conditions.

The fact that the sulphonic acid group-containing carbanoylonium compound described here prove virtually odourless under the conditions mentioned also means that they can be processed without danger of exceeding the maximum workplace concentration.

The compounds used in the fashion according to the invention are expediently added, preferably in the form of aqueous or alcoholic solutions, to the reagent coatings to be hardened immediately before pouring. The addition just before pouring is necessary since the compounds react very quickly with gelatin or the other proteins conventionally used in diagnostic chemistry. After the compounds have been added, the pouring solutions should be poured within a few minutes. The rate at which the hardening reaction proceeds depends primarily on the concentration of the proteins in the pouring solution.

In contrast to the derivatives of unsubstituted or lower alkyl radical-substituted pyridine, which cause a considerable exposure to odour on pouring and during drying, the compounds according to the invention crosslink the gelatin without emission of gaseous products.

The MWC values (also called threshold limit values in the USA) indicate the maximum workplace concentration, of a gaseous-vaporous or dusty material in the air of a workroom, which does not impair the health of those employed in the workroom during a daily working period of 8 hours, even on action over years.

A further possibility for using the compounds is first to pour the non-hardened pouring solutions and then to coat the coatings thus produced with a solution of the hardening compounds.

Apart from gelatin, gelatin derivatives, proteins and protein derivatives, the coatings may contain water-soluble highly polymeric compounds, particularly polyvinyl alcohol, sodium polyacrylate and other carboxyl group-containing homo- or copolymers, furthermore polyvinylpyrrolidone, polyacrylamide or high molecular weight natural substances, such as dextrans, dextrins, starch ether, alginic acid or alginic acid derivatives.

The concentrations to be used of the hardening agents according to the invention may vary within broad limits and essentially depend on the hardening compound used.

Amounts from 0.1 - 10 % by weight and preferably 0.2 - 6 % by weight, relative to the dry weight of binding agent, produce good results.

As stated above, the hardening reaction between the compounds of the invention and the gelatin or the proteins commences immediately, so that the optimum degree of hardening is achieved approximately simultaneously with the drying of the coatings subsequent to the pouring or their processing.

The action of the hardening compounds is determined with the aid of the melting point of the coatings, which can be determined in the following fashion:

Half of the coating, poured on a substrate, is dipped in water which is continuously warmed to 100 ° C. The temperature at which the coating runs off the substrate (streak formation) is designated as melting point or melt-off point. The melting point of pure protein or gelatin coatings without hardening agents is not increased in any case according to this measurement method. Under these conditions, the melt-off point is at 30-35 ° C.

The compounds according to the invention react rapidly with proteins in a surprising fashion and thus make it possible to harden protein-containing materials to an optimum degree in a very short time. This unexpected action of the compound is of particular importance for the hardening of diagnostic materials which contain proteins or protein derivatives as binding agents. The desired degree of hardening can be set, in an easily checkable fashion, virtually during the preparation of the materials without relatively long storage times and without the uncertainties which go along with these of uncontrollable post-hardening needing to be accepted.

Example 9

A 10 % strength casein solution is prepared in water by adding sodium hydroxide solution. 0.1 g of tartrazine is added to 100 ml of solution as filter dyestuff. Before pouring, various samples of the solution of 3 % by weight of each of the compounds 22, 19, 20, 21, 24, 25, 30, 31, 37, 41, 44, 45, 50, 52, 53, 55 and 56, dissolved in water, are added at a pH of 7. The mixtures are poured onto glass plates, and hardened filter foils, which no longer dissolve in alkaline water, are obtained after drying.

## Claims

**1.** Use of a hardened dry chemical reagent coatings for clinical chemical measurements having a matrix based on protein and/or polymer, characterized in that the matrix is hardened using a carboxyl group-activating crosslinking agent.

**2.** Use according to Claim 1 wherein the hardened reagent coatings were hardened using a carboxyl group-activating crosslinking agent from the group comprising
a) carbamoyl compounds of the general formula I

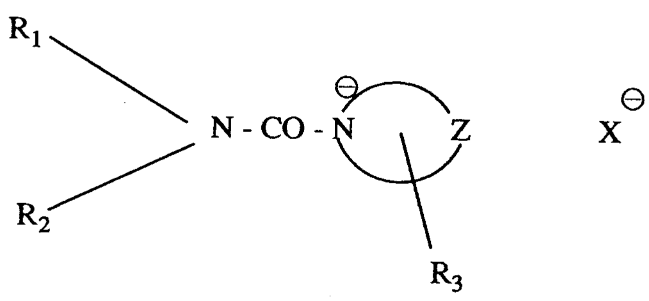

(I)

in which:
$R_1$ denotes an alkyl group, preferably having 1-3 C atoms, an aryl group which is optionally substituted by a $C_1$-$C_3$ alkyl radical or by halogen, for example phenyl which is optionally substituted by methyl, ethyl or propyl, Cl or Br, or denotes an aralkyl group, for example benzyl,

which may be substituted in the same fashion as the aryl group,

$R_2$ has the same meaning as $R_1$ or represents a divalent, optionally substituted alkylene, arylene, aralkylene or alkyl-aryl-alkyl radical, for example an ethylene-propylene, phenylene or xylylene radical, which is bonded to a further carbamoylammonium group of the above general formula via its second bond, or

$R_1$ and $R_2$, together, form the group of atoms which is necessary for completion of an optionally substituted piperidine, piperazine or morpholine ring, it being possible for the ring to be substituted, for example by an alkyl group having 1-3 C atoms or by halogen, such as Cl or Br,

$R_3$ denotes $-NR_4-CO-R_5$
: $R_4$ denotes H or alkyl (1-4 C)
: $R_5$ denotes H, alkyl (1-4 C) or $NR_6R_7$, and
: $R_6$ and $R_7$ denote H or alkyl ($C_1$-$C_4$)

$R_3$ denotes $-(CH_2)_m-NR_8R_9$
: $R_8$ denotes $-CO-R_{10}$
: $R_9$ denotes H or alkyl ($C_1$-$C_4$)
: $R_{10}$ denotes $NR_{11}R_{12}$
: $R_{11}$ denotes alkyl ($C_1$-$C_4$) or aryl
: $R_{12}$ denotes H, alkyl or aryl, and
: m denotes 1-3

$R_3$ denotes $-(CH_2)_n-CONR_{13}R_{14}$
: $R_{13}$ denotes H, alkyl ($C_1$-$C_4$) or aryl
: $R_{14}$ denotes H or alkyl ($C_1$-$C_4$), or
: $R_{13}$ and $R_{14}$ together form the group of atoms which is necessary for completion of a 5- or 6-membered aliphatic ring and
: n denotes 0-3

$R_3$ denotes

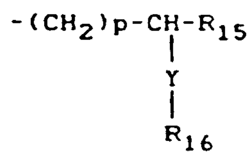

: $R_{15}$ denotes H or alkyl ($C_1$-$C_4$) which is optionally substituted by halogen
: Y denotes -O- or $-NR_{17}-$,
: $R_{16}$ denotes H, alkyl or $-CO-R_{18}$ or $-CO-NHR_{19}$
: $R_{17}$, $R_{18}$ and $R_{19}$ denote H or alkyl ($C_1$-$C_4$) and
: p denotes 2-3

Z denotes the C atoms which are necessary for completion of a 5- or 6-membered aromatic heterocyclic ring, optionally with fused benzene ring, and $X^-$ denotes an acid anion,

or

b) carbamoylammonium compound of the general formula II

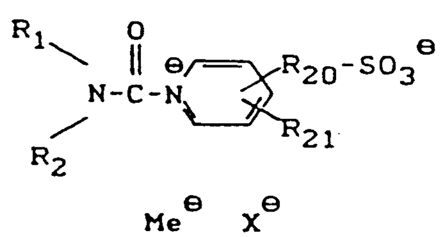

(II)

in which

$R_1$ and $R_2$ have the meaning stated in the case of the carbamoyl compounds of the formula I, and

$R_{20}$ represents methylene, ethylene, propylene, or a single chemical bond,

$R_{21}$ represents hydrogen, methyl or ethyl,

$X^{\ominus}$ represents an acid anion, such as $halogen^-$, $BF_4^{\ominus}$, $NO_3^{\ominus}$, $SO_4^{\ominus}$, $ClO_4^{\ominus}$ or $CH_3OSO_3^{\ominus}$

and

$Me^{+}$ represents an alkali metal cation,

or

c) carbamoylammonium compound of the general formula III

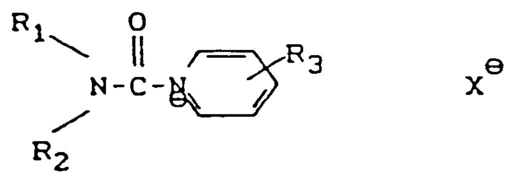

III

in which

$R_1$ and $R_2$ (which may be identical or different) in each case denote an alkyl group having 1 to 10 carbon atoms an aryl group having 6 to 15 carbon atoms or an aralkyl group having 7 to 15 carbon atoms (for example benzyl, phenethyl ), or they may be combined with one another to form a heterocyclic ring together with the nitrogen atom;

$R_3$ denotes a hydrogen atom, a halogen atom, a carbamoyl group, a sulpho group, a halogen atom, a carbamoyl group, a sulpho group, a ureido group, an alkoxy group having 1 to 10 carbon atoms or an alkyl group having 1 to 10 carbon atoms, or, when $R_3$ represents an alkyl or an alkoxy group, as described above, these groups may in each case be further substituted by a halogen atom, a carbamoyl group, a sulpho group or a ureido group;

$X^-$, in the formula III, represents an anion and acts as a counterion for the N-carbamoyl-pyridinium ion with formation of a salt, or it can be absent therein when

$R_3$ contains a sulpho group as component, and an intramolecular salt is thus formed, or

d) carbamoyloxoammonium compounds of the formula IV

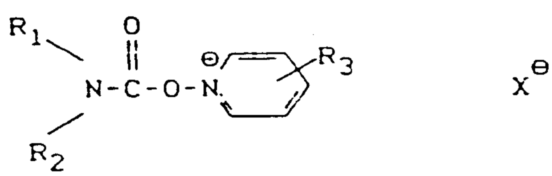

IV

in which

$R_1$, $R_2$, $R_3$ and $X^-$ have the same meanings as stated in the formula III,

or

e) compounds of the formula V

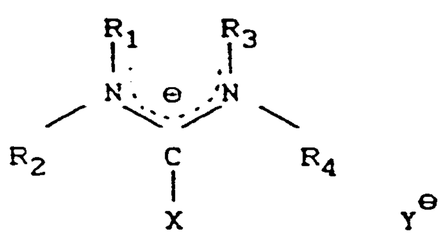

V

in which

$R_1$, $R_2$, $R_3$ and $R_4$ (which may be identical or different) in each case denote an alkyl group having 1 to 20 carbon atoms, an aralkyl group having 6 to 20 carbon atoms, or an aryl group having 5 to 20 carbon atoms, it being possible for these groups to be further substituted, or two groups, selected from $R_1$, $R_2$, $R_3$ and $R_4$, are combined with one another to form a ring, X denotes a group which can be cleaved off from the compound of the formula V by reaction with a nucleophilic reagent, suitable examples comprising a halogen atom, a sulphonyloxy group and a 1-pyridiniumyl group,

$Y^-$ denotes an anion, suitable examples comprising a halide ion, a sulphonate ion, a sulphuric acid ion, $ClO_4^-$, $BF_4^-$ and $PF_6^-$,

or

f) compounds of the formula VI

$R_1$-N=C=N-$R_2$ VI

in which
$R_1$ denotes an alkyl group having 1 to 10 carbon atoms, a cycloalkyl group having 5 to 8 carbon atoms, an alkoxyalkyl group having 3 to 10 carbon atoms, or an aralkyl group having 7 to 15 carbon atoms, or $R_2$, additionally to the groups defined as $R_1$, represents a group of the following formula:

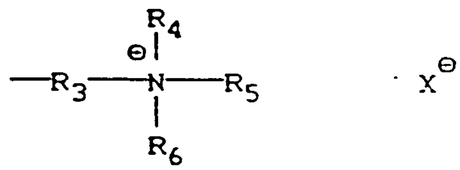

in which
$R_3$ represents an alkylene group having 2 to 4 carbon atoms; and
$R_4$ and $R_5$ (which may be identical or different) in each case denote an alkyl group having 1 to 6 carbon atoms, or it is also favourable when they are combined with one another to form a heterocyclic ring together with a nitrogen atom,
$R_6$ denotes an alkyl group having 1 to 6 carbon atoms, which is preferably substituted by a substituent from the group comprising the unsubstituted and substituted carbamoyl groups or the supho group,
$X^-$ denotes an anion, such as halide ions, sulphonate ions, sulphuric acid ions, $ClO_4^-$, $BF_4^-$ and $PF_6^-$,
or
g) halogenopyridinium compounds of the formula VII

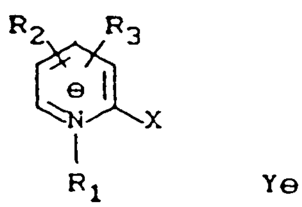

VII

in which
$R_1$ denotes an alkyl group having 1 to 10 carbon atoms, an aryl group having 6 to 15 carbon atoms, or an aralkyl group having 7 to 15 carbon atoms, which may in each case be substituted,
$R_2$ and $R_3$ (which may be identical or different) denote a hydrogen atom, a halogen atom, an acylamido group, a nitro group, a carbamoyl group, a ureido group, an alkoxy group, an alkyl group, an alkenyl group, an aryl group, an aralkyl group or an aryl group, or
$R_2$ and $R_3$ are combined with one another to form a condensed ring together with the pyridinium ring structure.
X denotes a group which is cleaved off from the compound represented by the formula VII by reaction with a nucleophilic reagent, such as, for example, halogen atoms, the sulphonyloxy group, or groups which are represented by

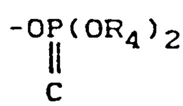 or 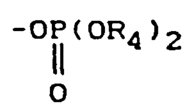 ,

in which
$R_4$ represents an alkyl group or an aryl group, and
$Y^-$ represents an anion, such as halide ions, sulphonate ions, sulphuric acid ions, $ClO_4^-$, $BF_4^-$, $PF_6^-$

or

h) sulphonylaminopiperidinum salts of the formula VIII

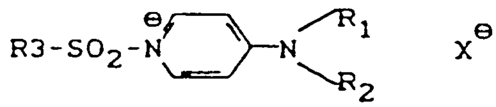

VIII

in which
$R_1$ and $R_2$ have the same meanings as defined for $R_1$ and $R_2$ in the formula I, and
$R_3$ denotes an alkyl group having 1 to 10 carbon atoms, an aryl group having 6 to 15 carbon atoms or an aralkyl group having 7 to 15 carbon atoms, and
$X^-$ denotes an anion, such as halide ions, sulphonate ions, sulphuric acid ions, $ClO_4^-$, $BF_4^-$, $PF_6^-$.

**3.** Use according to claim 1 and 2 wherein the hardened reagent coatings contain gelatin, gelatin derivatives, casein and/or zein as binding agent.

**4.** Use according to any of Claims 1 - 3 wherein the hardened reagent coatings are coatings, in which the amount of crosslinking agent is 0.2 to 40 % by weight, relative to the weight of the binding agent.

**5.** Use according to any of Claims 1 - 4 wherein the hardened reagent coatings are coatings containing reagents from the group comprising enzymes, coenzymes, effectors, indicators, substrates, antigens, antibodies, stabilizers, wetting agents and buffers.

**6.** Process for the preparation of hardened reagent coatings to be used according to Claims 1 to 5, characterized in that the crosslinking agent is added to a pouring solution which contains a binding agent, and the resulting solution is poured to form a coating and is subsequently dried.

**7.** Process for the preparation of hardened reagent coatings to be used according to Claims 1 to 5, characterized in that

a) a pouring solution which contains a biding agent is poured to form a binding-agent coating, and

b) the crosslinking agent, in the form of a solution, is subsequently brought into contact with the binding-agent coating in order to effect the hardening.

**8.** Process according to Claim 7, characterized in that the binding agent coating is coated with a 0.2 to 5 % strength solution of the crosslinking agent.

**9.** Process, according to Claims 7 to 9, in which the crosslinking agent is used as an alcoholic, aqueous or aqueous-alcoholic solution.

**Revendications**

**1.** Utilisation de revêtements réactifs chimiques secs durcis pour des mesures chimiques cliniques, comprenant une matrice à base d'une protéine et/ou d'un polymère, caractérisée en ce que la matrice est durcie au moyen d'un agent de réticulation activateur de groupe carboxyle.

**2.** Utilisation suivant la revendication 1, dans laquelle les revêtements réactifs durcis ont été durcis au moyen d'un agent de réticulation activateur du groupe carboxyle, choisi entre

a) des composés carbamoyliques de formule générale I

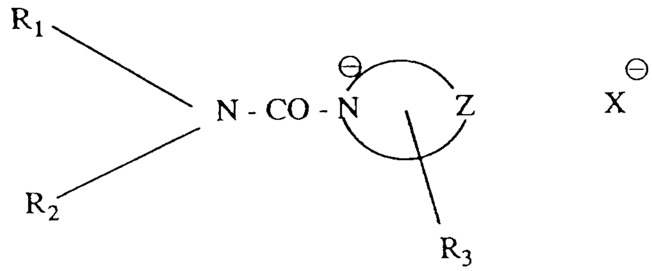 (I)

dans laquelle

$R_1$ désigne un groupe alkyle, ayant de préférence 1 à 3 atomes de carbone, un groupe aryle qui est facultativement substitué par un radical alkyle en $C_1$ à $C_3$ ou par un halogène, par exemple un groupe phényle qui est facultativement substitué par un radical méthyle, éthyle ou propyle, Cl ou Br, ou désigne un groupe aralkyle, par exemple benzyle, qui peut être substitué de la même façon que le groupe aryle,

$R_2$ a la même définition que $R_1$ ou représente un radical alkylène, arylène, aralkylène ou alkylarylalkyle divalent facultativement substitué, par exemple un radical éthylènepropylène, phénylène ou xylylène, qui est lié à un autre groupe carbamoylammonium de la formule générale ci-dessus par l'intermédiaire de sa seconde liaison, ou bien

$R_1$ et $R_2$ forment conjointement le groupe d'atomes qui est nécessaire pour compléter un noyau de pipéridine, de pipérazine ou de morpholine facultativement substitué, le noyau pouvant être substitué par exemple par un groupe alkyle ayant 1 à 3 atomes de carbone ou par un halogène tel que Cl ou Br,

| | |
|---|---|
| $R_3$ représente $-NR_4-CO-R_5$ | $R_4$ représente H ou un groupe alkyle en $C_1$ à $C_4$<br>$R_5$ représente H, un groupe alkyle en $C_1$ à $C_4$ ou un groupe $NR_6N_7$, et<br>$R_6$ et $R_7$ représentent H ou un groupe alkyle en $C_1$ à $C_4$ |
| $R_3$ représente un groupe $-(CH_2)_m-NR_8R_9$ | $R_8$ représente $-CO-R_{10}$<br>$R_9$ représente H ou un groupe alkyle en $C_1$ à $C_4$<br>$R_{10}$ est un groupe $NR_{11}R_{12}$<br>$R_{11}$ est un groupe alkyle en $C_1$ à $C_4$ ou aryle<br>$R_{12}$ représente H, un groupe alkyle ou aryle et<br>m a une valeur de 1 à 3 |
| $R_3$ représente un groupe $-(CH_2)_n-CONR_{13}R_{14}$ | $R_{13}$ représente H, un groupe alkyle en $C_1$ à $C_4$ ou aryle<br>$R_{14}$ représente H ou un groupe alkyle en $C_1$ à $C_4$, ou bien<br>$R_{13}$ et $R_{14}$ forment conjointement le groupe d'atomes qui est nécessaire pour compléter un noyau aliphatique pentagonal ou hexagonal et<br>n a une valeur de 0 à 3 |
| $R_3$ représente | 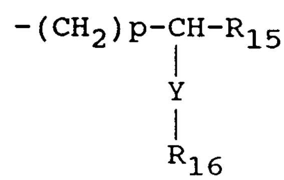<br>$R_{15}$ représente H ou un groupe alkyle en $C_1$ à $C_4$ qui est facultativement substitué par un halogène |

Y représente -O- ou $-NR_{17}-$
$R_{16}$ représente H, un groupe alkyle ou un groupe $-CO-R_{18}$ ou $-CO-NHR_{19}$
$R_{17}$, $R_{18}$ et $R_{19}$ représentent H ou un groupe alkyle en $C_1$ à $C_4$ et
p a une valeur de 2-3

Z désigne les atomes de carbone qui sont nécessaires pour compléter un noyau hétérocyclique aromatique, pentagonal ou hexagonal, facultativement avec un noyau benzénique condensé, et $X^{\ominus}$ est un anion d'acide, ou bien

b) un composé de carbamoylammonium de formule générale II

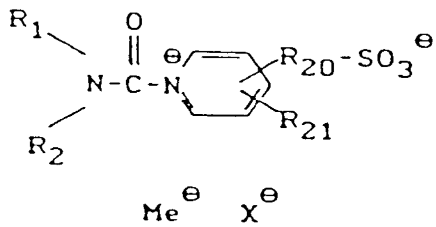

(II)

dans laquelle
$R_1$ et $R_2$ ont la définition indiquée dans le cas des composés carbamoyle de formule I, et
$R_{20}$ représente un groupe méthylène, éthylène, propylène ou une simple liaison chimique,
$R_{21}$ représente l'hydrogène, un groupe méthyle ou éthyle
$X^{\ominus}$ représente un anion d'acide tel qu'halogène$^{\ominus}$, $BF_4^{\ominus}$, $NO_3^{\ominus}$, $SO_4^{\ominus}$, $ClO_4^{\ominus}$ ou $CH_3OSO_3^{\ominus}$ et
$Me^{\oplus}$ représente un cation de métal alcalin, ou bien

c) un composé de carbamoylammonium de formule générale III

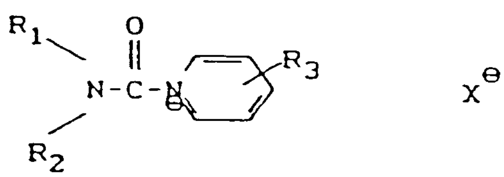

III

dans laquelle
$R_1$ et $R_2$ (qui peuvent être identiques ou différents) désignent dans chaque cas un groupe alkyle ayant 1 à 10 atomes de carbone, un groupe aryle ayant 6 à 15 atomes de carbone ou un groupe aralkyle ayant 7 à 15 atomes de carbone (par exemple benzyle, phénéthyle) ou bien ils peuvent s'associer pour former un noyau hétérocyclique avec l'atome d'azote ;
$R_3$ représente un atome d'hydrogène, un atome d'halogène, un groupe carbamoyle, un groupe sulfo, un atome d'halogène, un groupe carbamoyle, un groupe sulfo, un groupe uréido, un groupe alkoxy ayant 1 à 10 atomes de carbone ou un groupe alkyle ayant 1 à 10 atomes de carbone, ou bien, lorsque $R_3$ représente un groupe alkyle ou un groupe alkoxy, comme décrit ci-dessus, ces groupes peuvent dans chaque cas être encore substitués par un atome d'halogène, un groupe carbamoyle, un groupe sulfo ou un groupe uréido ;
$X^{\ominus}$, dans la formule III, représente un anion et agit comme un ion complémentaire pour l'ion N-carbamoyl-pyridinium avec formation d'un sel, ou bien il peut être absent lorsque
$R_3$ contient un groupe sulfo comme composant, et un sel intramoléculaire est ainsi formé, ou bien

d) des composés de carbamoyloxoammonium de formule IV

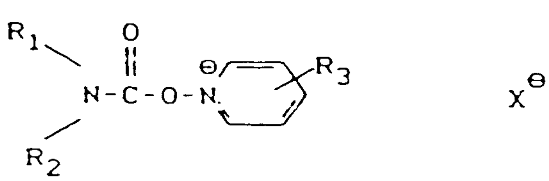

IV

dans laquelle
$R_1$, $R_2$, $R_3$ et $X^{\ominus}$ ont les mêmes définitions que celles qui ont été indiquées pour la formule III, ou bien
e) des composés de formule V

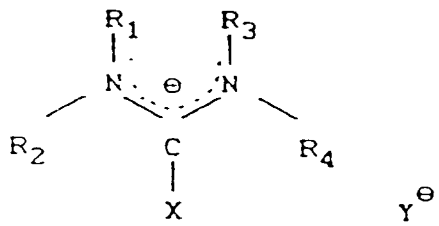

V

dans laquelle
$R_1$, $R_2$, $R_3$ et $R_4$ (qui peuvent être identiques ou différents) désignent dans chaque cas un groupe alkyle ayant 1 à 20 atomes de carbone, un groupe aralkyle ayant 6 à 20 atomes de carbone ou un groupe aryle ayant 5 à 20 atomes de carbone, ces groupes pouvant encore être substitués, ou bien deux groupes choisis entre $R_1$, $R_2$, $R_3$ et $R_4$ s'associent ensemble pour former un noyau, X désigne un groupe qui peut être scindé du composé de formule V par réaction avec un réactif nucléophile, des exemples convenables comprenant un atome d'halogène, un groupe sulfonyloxy et un groupe 1-pyridiniumyle,
$Y^{\ominus}$ représente un anion, des exemples convenables comprenant un ion halogénure, un ion sulfonate, un ion acide sulfurique, $ClO_4^-$, $BF_4^-$ et $PF_6^-$
ou bien
f) des composés de formule VI

$R_1$-N=C=N-$R_2$ VI

dans laquelle
$R_1$ désigne un groupe alkyle ayant 1 à 10 atomes de carbone, un groupe cycloalkyle ayant 5 à 8 atomes de carbone, un groupe alkoxyalkyle ayant 3 à 10 atomes de carbone ou un groupe aralkyle ayant 7 à 15 atomes de carbone, ou bien $R_2$, en plus des groupes définis comme $R_1$, représente un groupe de formule suivante :

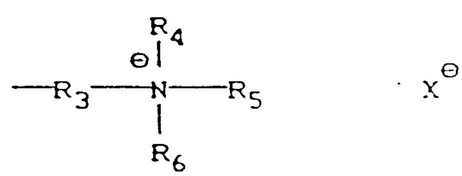

dans laquelle
$R_3$ représente un groupe alkylène ayant 2 à 4 atomes de carbone ; et
$R_4$ et $R_5$ (qui peuvent être identiques ou différents) désignent dans chaque cas un groupe alkyle ayant 1 à 6 atomes de carbone, ou bien il peut aussi être avantageux qu'ils s'associent ensemble pour former un noyau hétérocyclique conjointement avec un atome d'azote,
$R_6$ désigne un groupe alkyle ayant 1 à 6 atomes de carbone, qui porte de préférence un substituant choisi entre des groupes carbamoyle non substitués et substitués ou le groupe sulfo,
$X^{\ominus}$ désigne un anion tel que des ions halogénure, des ions sulfonate, des ions acide sulfurique, $ClO_4^-$, $BF_4^-$ et $PF_6^-$,
ou bien
g) des composés d'halogéno-pyridinium de formule VII

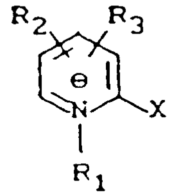

VII

$Y^{\ominus}$

dans laquelle

$R_1$ désigne un groupe alkyle ayant 1 à 10 atomes de carbone, un groupe aryle ayant 6 à 15 atomes de carbone ou un groupe aralkyle ayant 7 à 15 atomes de carbone, qui peut dans chaque cas être substitué,

$R_2$ et $R_3$ (qui peuvent être identiques ou différents) désignent un atome d'hydrogène, un atome d'halogène, un groupe acyl-amido, un groupe nitro, un groupe carbamoyle, un groupe uréido, un groupe alkoxy, un groupe alkyle, un groupe alcényle, un groupe aryle, un groupe aralkyle ou un groupe aryle, ou bien $R_2$ et $R_3$ s'associent pour former un noyau condensé conjointement avec la structure de noyau pyridinium,

X désigne un groupe qui est scindé du composé représenté par la formule VII par réaction avec un réactif nucléophile tel que par exemple des atomes d'halogènes, le groupe sulfonyloxy ou des groupes qui sont représentés par

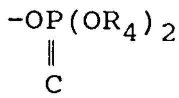 ou 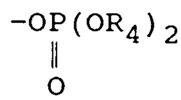 ,

où

$R_4$ représente un groupe alkyle ou un groupe aryle et

$Y^{\ominus}$ représente un anion tel que des ions halogénure, des ions sulfonate, des ions acide sulfurique, $ClO_4^-$, $BF_4^-$, $PF_6^-$

h) des sels de sulfonylaminopipéridinium de formule :

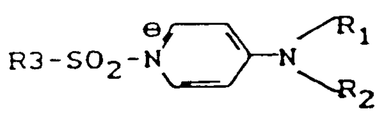

$X^{\ominus}$ VIII

dans laquelle

$R_1$ et $R_2$ ont les définitions qui ont été données pour $R_1$ et $R_2$ dans la formule I et

$R_3$ désigne un groupe alkyle ayant 1 à 10 atomes de carbone, un groupe aryle ayant 6 à 15 atomes de carbone ou un groupe aralkyle ayant 7 à 15 atomes de carbone, et

$X^{\ominus}$ désigne un anion tel que des ions halogénure, des ions sulfonate, des ions acide sulfurique, $ClO_4^-$, $BF_4^-$, $PF_6^-$.

3. Utilisation suivant les revendications 1 et 2, dans laquelle les revêtements réactifs durcis contiennent de la gélatine, des dérivés de gélatine, de la caséine et/ou de la zéine comme agent liant.

4. Utilisation suivant l'une quelconque des revendications 1 à 3, dans laquelle les revêtements réactifs durcis sont des revêtements dans lesquels la quantité d'agent de réticulation va de 0,2 à 40% en poids, par rapport au poids de l'agent liant.

5. Utilisation suivant l'une quelconque des revendications 1 à 4, dans laquelle les revêtements réactifs durcis sont des revêtements contenant des réactifs choisis dans le groupe comprenant des enzymes, des coenzymes, des effecteurs, des indicateurs, des substrats, des antigènes, des anticorps, des agents stabilisants, des agents mouillants et des tampons.

6. Procédé de préparation de revêtements réactifs durcis destinés à être utilisés suivant les revendications 1 à 5, caractérisé en ce que l'agent de réticulation est ajouté à une solution à couler qui contient un agent liant, et la solution résultante est coulée pour former un revêtement qui est ensuite séché.

7. Procédé de préparation de revêtements réactifs durcis destinés à être utilisés conformément aux revendications 1 à 5, caractérisé en ce que :
a) une solution à couler qui contient un agent liant est coulée pour former un revêtement renfermant ledit agent et
b) l'agent de réticulation, sous forme d'une solution, est ensuite mis en contact avec le revêtement contenant le liant de manière à effectuer le durcissement.

8. Procédé suivant la revendication 7, caractérisé en ce que le revêtement contenant l'agent liant est revêtu d'une solution à 0,2-5% de l'agent de réticulation.

9. Procédé suivant les revendications 7 à 9, dans lequel l'agent de réticulation est utilisé sous forme d'une solution alcoolique, aqueuse ou aqueuse-alcoolique.

**Patentansprüche**

1. Verwendung von gehärteten, trockenen, chemischen Reagenzschichten für klinische chemische Messungen mit einer Matrix auf Grundlage eines Proteins und/oder Polymers, dadurch **gekennzeichnet,** dass die Matrix unter Verwendung eines carboxylgruppenaktivierenden Vernetzers gehärtet wird.

2. Verwendung nach Anspruch 1, worin die gehärteten Reagensschichten unter Verwendung eines carboxylgruppenaktivierenden Vernetzers aus der Gruppe, bestehend aus
(a) Carbamoylverbindungen der allgemeinen Formel (I)

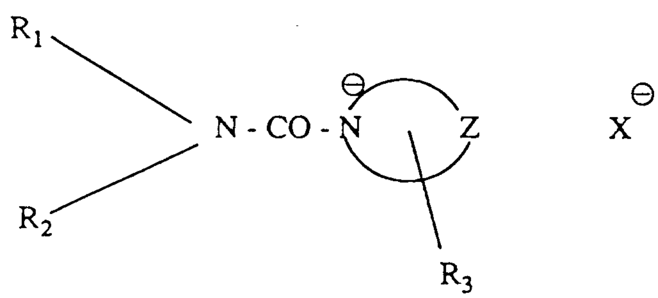

(I)

worin
$R_1$ eine Alkylgruppe bedeutet, vorzugsweise mit 1 bis 3 C-Atomen, eine Arylgruppe, die wahlweise durch ein $C_{1-3}$-Alkylradikal oder Halogen substituiert ist, z.B. Phenyl, welches wahlweise durch Methyl, Ethyl oder Propyl, Chlor oder Brom substituiert ist, oder eine Aralkylgruppe bedeutet, z.B. Benzyl, die in derselben Weise wie die Arylgruppe substituiert sein kann;
$R_2$ hat dieselbe Bedeutung wie $R_1$ oder stellt ein divalentes, wahlweise substituiertes Alkylen, Arylen, Aralkylen oder Alkyl-aryl-alkyl-radikal dar, z.B. ein Ethylen-propylen-, Phenylen- oder Xylylenradikal, das an eine weitere Carbamoylammoniumgruppe der obigen allgemeinen Formel über seine zweite Bindung gebunden ist; oder
$R_1$ und $R_2$ bilden zusammen eine Gruppe von Atomen, die zur Vervollständigung eines wahlweise substituierten Piperidin-, Piperazin- oder Morpholinringes nötig ist, wobei der Ring substituiert sein kann, z.B. durch eine Alkylgruppe mit 1 bis 3 C-Atomen, oder durch Halogen, wie Chlor oder Brom;
$R_3$ bedeutet $-NR_4-CO-R_5$ [$R_4$ bedeutet H oder Alkyl (1-4 C), $R_5$ bedeutet H, Alkyl (1-4 C) oder $NR_6R_7$ und $R_6$ und $R_7$ bedeuten H oder Alkyl ($C_{1-4}$)],
$R_3$ bedeutet $-(CH_2)_m-NR_8R_9$ [$R_8$ bedeutet $-CO-R_{10}$, $R_9$ bedeutet H oder Alkyl ($C_{1-4}$), $R_{10}$ bedeutet $NR_{11}R_{12}$, $R_{11}$ bedeutet Alkyl ($C_{1-4}$) oder Aryl, $R_{12}$ bedeutet H, Alkyl oder Aryl, und m bedeutet 1 bis 3],
$R_3$ bedeutet $-(CH_2)_n-CONR_{13}R_{14}$ [$R_{13}$ bedeutet H, Alkyl ($C_{1-4}$) oder Aryl, $R_{14}$ bedeutet H oder Alkyl ($C_{1-4}$) oder $R_{13}$ und $R_{14}$ bilden zusammen die Gruppe von Atomen, die notwendig sind zur Vervollständigung eines 5- oder 6-gliedrigen aliphatischen Ringes, und n bedeutet 0 bis 3],
$R_3$ bedeutet

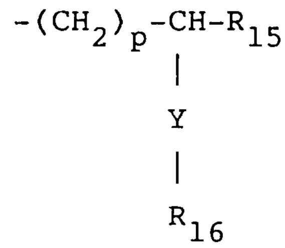

[$R_{15}$ bedeutet H oder Alkyl ($C_{1-4}$), welches wahlweise durch Halogen substituiert ist, Y bedeutet -O- oder -$NR_{17}$-, $R_{16}$ bedeutet H, Alkyl oder -CO-$R_{18}$ oder -CO-$NHR_{19}$, $R_{17}$, $R_{18}$ und $R_{19}$ bedeuten H oder Alkyl ($C_{1-4}$) und p bedeutet 2 bis 3];
Z bedeutet die C-Atome, die notwendig sind zur Vervollständigung eines 5- oder 6-gliedrigen aromatischen, heterozyklischen Ringes, der wahlweise mit einem Benzolring fusioniert ist, und $X^-$ bedeutet ein Säureanion;
oder
(b) Carbamoylammoniumverbindung der allgemeinen Formel (II)

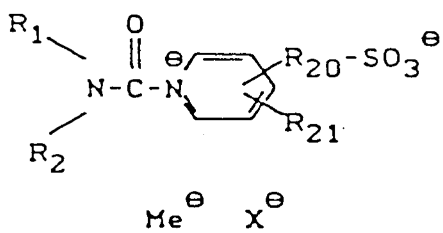

(II)

in der $R_1$ und $R_2$ die Bedeutung im Falle der Carbamoylverbindungen der Formel (I) haben; und
$R_{20}$ stellt eine Methylen-, Ethylen-, Propylen- oder eine Einfachbindung dar;
$R_{21}$ stellt Wasserstoff, Methyl oder Ethyl dar;
$X^-$ stellt ein Säureanion, wie z.B. $Halogen^-$, $BF_4^-$ , $NO_3^-$ , $SO_4^-$ , $ClO_4^-$ oder $CH_3OSO_3^-$ dar; und
$Me^+$ repräsentiert ein Alkalimetallion;
oder
(c) Carbamoylammoniumverbindung der allgemeinen Formel (III)

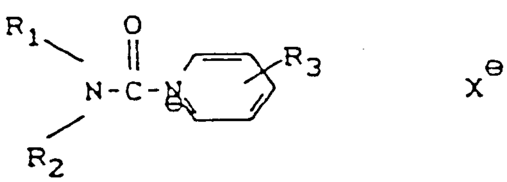

(III)

in der $R_1$ und $R_2$ (welche gleich oder verschieden sein können) jeweils eine Alkylgruppe mit 1 bis 10 Kohlenstoffatomen, eine Arylgruppe mit 6 bis 15 Kohlenstoffatomen, oder eine Aralkylgruppe mit 7 bis 15 Kohlenstoffatomen (z.B. Benzyl, Phenethyl) bedeuten können, oder sie können miteinander verbunden sein unter Ausbildung eines heterozyklischen Ringes zusammen mit einem Stickstoffatom;
$R_3$ bedeutet ein Wasserstoffatom, ein Halogenatom, eine Carbamoylgruppe, eine Sulfogruppe, ein Halogenatom, eine Carbamoylgruppe, eine Sulfogruppe, eine Ureidogruppe, eine Alkoxygruppe mit 1 bis 10 Kohlenstoffatomen oder eine Alkylgruppe mit 1 bis 10 Kohlenstoffatomen; oder
wenn $R_3$ eine Alkyl- oder Alkoxygruppe darstellt, wie oben beschrieben, können diese Gruppen jeweils weiter durch ein Halogenatom, eine Carbamoylgruppe, eine Sulfogruppe oder eine Ureidogruppe substituiert sein;
$X^-$ in der Formel (III) stellt ein Anion dar und fungiert als Gegenion für das N-Carbamoylpyridiniumion unter Bildung eines Salzes oder es kann abwesend sein, wenn $R_3$ eine Sulfogruppe

als Bestandteil enthält und daher ein intramolekulares Salz gebildet wird; oder
(d) Carbamoyloxoammonium-Verbindungen der Formel (IV)

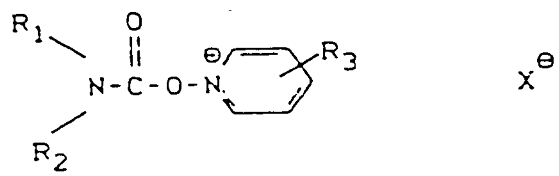

(IV)

in der $R_1$, $R_2$, $R_3$ und $X^-$ dieselben Bedeutungen wie in der Formel (III) haben; oder
(e) Verbindungen der Formel (V)

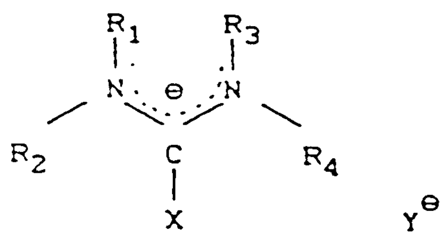

(V)

in der $R_1$, $R_2$, $R_3$ und $R_4$ (welche gleich oder verschieden sein können) jeweils eine Alkylgruppe mit 1 bis 20 Kohlenstoffatomen, eine Aralkylgruppe mit 6 bis 20 Kohlenstoffatomen oder eine Arylgruppe mit 5 bis 20 Kohlenstoffatomen bedeuten, wobei es für diese Gruppen möglich ist, zusätzlich substituiert vorzuliegen, oder zwei Gruppen, ausgewählt aus $R_1$, $R_2$, $R_3$ und $R_4$ sind miteinander unter Bildung eines Ringes verbunden, X bedeutet eine Gruppe, die von der Verbindung der Formel (V) durch Reaktion mit einem nukleophilen Reagens abgespaltet werden kann, geeignete Beispiele hierfür umfassen ein Halogenatom, eine Sulfonyloxygruppe und eine 1-Pyridiniumylgruppe,
$Y^-$ bedeutet ein Anion, geeignete Beispiele umfassen ein Halogenidion, ein Sulfonation, ein Sulfation, $ClO_4^-$, $BF_4^-$ und $PF_6^-$; oder
(f) Verbindungen der Formel (VI)

$R_1$-N = C = N-$R_2$ (VI)

in der $R_1$ eine Alkylgruppe mit 1 bis 10 Kohlenstoffatomen, eine Cycloalkylgruppe mit 5 bis 8 Kohlenstoffatomen, eine Alkoxyalkylgruppe mit 3 bis 10 Kohlenstoffatomen, oder eine Aralkylgruppe mit 7 bis 15 Kohlenstoffatomen bedeutet, oder $R_2$ zusätzlich zu den Gruppen, wie für $R_1$ definiert, eine Gruppe der folgenden Formel bedeutet

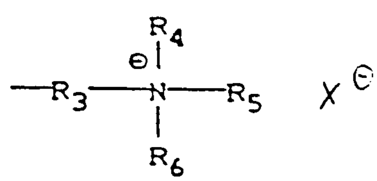

in der $R_3$ eine Alkylgruppe mit 2 bis 4 Kohlenstoffatomen; und
$R_4$ und $R_5$ (die gleich oder verschieden sein können) jeweils eine Alkylgruppe mit 1 bis 6 Kohlenstoffatomen bedeuten, oder es ist ebenfalls bevorzugt, wenn sie zusammen mit einem Stickstofatom miteinander verbunden sind unter Ausbildung eines heterozylischen Ringes, $R_6$ bedeutet eine Alkylgruppe mit 1 bis 6 Kohlenstoffatomen, die vorzugsweise durch einen Substituenten der Gruppe umfassend die unsubstituierten und substituierten Carbamoylgruppen oder Sulfogruppen substituiert;
$X^-$ bedeutet ein Anion, wie z.B. Halogenidion, Sulfonation, Sulfation, $ClO_4^-$, $BF_4^-$ und $PF_6^-$, oder
(g) Halogenpyridiniumverbindungen der Formel (VII)

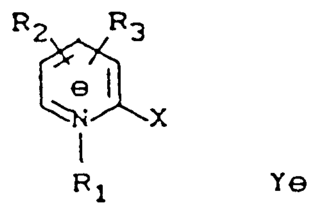

in denen $R_1$ eine Alkylgruppe mit 1 bis 10 Kohlenstoffatomen, eine Arylgruppe mit 6 bis 15 Kohlenstoffatomen oder eine Aralkylgruppe mit 7 bis 15 Kohlenstoffatomen bedeutet, die jeweils substituiert sein kann,

$R_2$ und $R_3$ (die gleich oder verschieden sein können) bedeuten ein Wasserstoffatom, ein Halogenatom, eine Acylamidogruppe, eine Nitrogruppe, eine Carbamoylgruppe, eine Ureidogruppe, eine Alkoxygruppe, eine Alkylgruppe, eine Alkenylgruppe, eine Arylgruppe, eine Aralkylgruppe oder eine Arylgruppe, oder

$R_2$ und $R_3$ sind miteinander unter Ausbildung eines kondensierten Ringes zusammen mit der Pyridiniumringstruktur verbunden,

X bedeutet eine Gruppe, die aus der Verbindung, dargestellt durch die Formel (VII) durch Reaktion mit einem nukleophilen Agens abgespalten weden kann, wie z.B. Halogenatom, Sulfonyloxygruppe oder Gruppen, die dargestellt sind durch

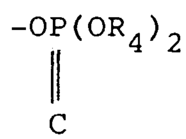

oder

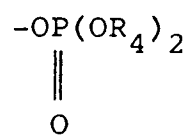

in denen $R_4$ eine Alkylgruppe oder eine Arylgruppe darstellt und

$Y^-$ ein Anion, wie z.B. ein Halogenid, Sulfonat, Sulfat, $ClO_4^-$, $BF_4^-$, $PF_6^-$, darstellt, oder

(h) Sulfonylaminopiperidiniumsalze der Formel (VIII)

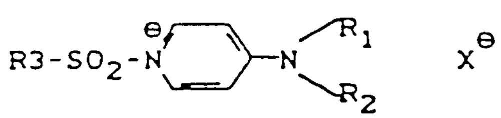

(VIII)

in der $R_1$ und $R_2$ dieselben Bedeutungen wie für $R_1$ und $R_2$ in der Formel (I) haben, und

$R_3$ eine Alkylgruppe mit 1 bis 10 Kohlenstoffatomen, eine Arylgruppe mit 6 bis 15 Kohlenstoffatomen, oder eine Aralkylgruppe mit 7 bis 15 Kohlenstoffatomen bedeutet, und

$X^-$ ein Anion, wie z.B. Halogenid, Sulfonat, Sulfat, $ClO_4^-$, $BF_4^-$, $PF_6^-$ bedeutet.

**3.** Verwendung nach einem der Ansprüche 1 und 2, worin die gehärteten Reagensschichten Gelatine, Gelatinederivate, Kasein und/oder Zein als Bindemittel enthalten.

**4.** Verwendung nach einem der Ansprüche 1 bis 3, worin die gehärteten Reagensschichten Schichten bilden, in denen die Menge des Vernetzers 0,2 bis 40 Gew.%, bezogen auf das Gewicht des Bindemittels, beträgt.

**5.** Verwendung nach einem der Ansprüche 1 bis 4, worin die gehärteten Reagensschichten Schichten bilden, die Reagenzien aus der folgenden Gruppe, umfassend Enzyme, Coenzyme, Effektoren, Indikatoren, Substrate, Antigene, Antikörper, Stabilisatoren, Benetzungsmittel und Puffer, enthalten.

**6.** Verfahren zur Herstellung gehärteter Reagensschichten zur Verwendung nach einem der Ansprüche 1 bis 5, ddurch **gekennzeichnet,** dass der Vernetzer in eine Ausgiesslösung gegeben wird, die einen Binder enthält, und die resultierende Lösung zur Ausbildung einer Schicht ausgegossen und anschliessend getrocknet wird.

7. Verfahren zur Herstellung von gehärteten Reagensschichten zur Verwendung nach einem der Ansprüche 1 bis 5, dadurch **gekennzeichnet,** dass
   (a) eine Ausgiesslösung, die einen Binder enthält, zur Ausbildung einer Binderschicht ausgegossen wird und
   (b) der Vernetzer in Form einer Lösung anschliessend mit der Binderschicht zur Aushärtung in Kontakt gebracht wird.

8. Verfahren nach Anspruch 7, dadurch **gekennzeichnet,** dass die Binderschicht mit einer 0,2 bis 5 % starken Lösung des Vernetzers beschichtet wird.

9. Verfahren nach Ansprüchen 7 bis 9, bei dem der Vernetzer als eine alkoholische wässrige oder wässrig-alkoholische Lösung eingesetzt wird.